# EUROPEAN PATENT APPLICATION

(11) **EP 4 159 242 A1**
(43) Date of publication of application: **05.04.2023**
(21) Application number: 21818271.5
(22) Date of filing: 01.06.2021
(51) Int. Cl.: A61K 47/64, A61P 35/00, A61P 27/02, A61P 19/02, A61P 29/00

(54) **DRUG CONJUGATE HAVING ENHANCED DRUG DELIVERY AND INTERNALIZATION EFFICIENCY**

(30) Priority: 01.06.2020 KR 20200065959
(71) Applicant: BIK Therapeutics Inc., Seongnam-si, Gyeonggi-do 13605 (KR); Seoul National University Hospital, Seoul 03080 (KR)
(72) Inventor: KIM, Sang Eun, Seoul 06320 (KR); LEE, Byung Chul, Hanam-si Gyeonggi-do 13014 (KR); YOUN, Hewon, Seoul 04196 (KR); CHOI, Ji Young, Seoul 05117 (KR); JUNG, Jae Ho, Seoul 04976 (KR); SONG, In Ho, Yongin-si Gyeonggi-do 16961 (KR)
(74) Representative: Schnappauf, Georg
(86) International application number: PCT/IB2021/054774
(87) International publication number: WO 2021/245539

(57) **Abstract**

The present invention relates to a unit drug conjugate wherein a binding group capable of binding to another unit drug conjugate is additionally linked to a unit drug conjugate in which a targeting substance, which binds specifically to target cells, and a drug, are linked together. When the unit drug conjugates according to the present invention are sequentially administered *in vivo,* complexes form a cluster by *in vivo* crosslinking, and the cluster promotes endocytosis of the drug conjugate, thereby significantly enhancing cellular internalization of the drug included in the drug conjugate.

## Description

### Technical Field

The present invention relates to a drug conjugate having enhanced drug delivery and cellular internalization efficiencies, and more particularly, to a unit drug conjugate wherein a binding group capable of binding to another drug conjugate is additionally linked to a drug conjugate in which a targeting substance, which binds specifically to target cells, and a drug, are linked together.

### Background Art

Endocytosis is a general term defining processes, by which a cell imports selected extracellular species, such as molecules, viruses, particles and microorganisms and target them to specific organelles within a cytoplasm. Endocytosis is generally divided into phagocytosis and pinocytosis, wherein pinocytosis may be sub-divided into macropinocytosis, clathrin-mediated endocytosis, caveolin-mediated endocytosis, clathrin- and caveolin-independent endocytosis.

The endocytic pathways may depend on the size of the endocytic vesicle, the nature of the cargo (ligands, receptors, and lipids), and the mechanism of vesicle formation (Conner S.D. et al., Nature 2003; 422:37-44). For example, macropinocytosis (> 1 µm) mainly delivers extracellular materials into cells by plasma membrane invagination, and clathrin-mediated endocytosis (~120 nm), caveolin-mediated endocytosis (∼60 nm), and clathrin- and caveolin-independent endocytosis (∼90 nm) introduce extracellular materials into cells via intracellular vesicles.

Endocytosis of extracellular materials plays an important role in target cellular internalization of drugs capable of specifically binding to molecules (e.g., receptors) widely expressed on cell membranes, in the fields of molecular biology such as virology or drug and gene delivery. In particular, as a method of effectively internalizing a drug into a target cell, efficient endocytosis may be induced by introducing a molecule (e.g., a ligand) capable of specifically binding to a molecule (e.g., a receptor) widely expressed on cell membranes (Marsh M. et al., Cell 2006; 124:729-40; Smith A.E. et al., Science 2004; 304:237-42). In this case, the receptor to which the drug has been bound is collected at the invagination site by surface diffusion. If this collection does not occur smoothly, endocytosis does not occur effectively within a short time.

Angiogenesis refers to the process in which new capillaries are formed from existing microvessels, and is known to play an important role in normal physiological processes such as embryogenesis, wound healing, and female reproductive cycling. However, there are diseases caused by failure of self-regulation of angiogenesis and abnormal growth of blood vessels. For example, abnormally excessive angiogenesis is known to play a critical role in growth and metastasis of tumors or cancers, and diseases such as diabetic retinopathy, age-related macular degeneration, rheumatoid arthritis, endometriosis, psoriasis, or chronic inflammation. Angiogenesis is also associated with coronary artery disease, stroke, myocardial infarction, ulcer or delayed wound healing.

Among these diseases, a tumor, particularly a malignant tumor (cancer) refers to a disease in which cells constituting the body divide irregularly by the action of internal or external factors and thus the cells themselves are out of a bodily control and proliferate unintentionally. Moreover, the tumor invades the surrounding tissues and metastasizes to other organs through blood vessels, lymphatic vessels, and the like and grows. At this time, angiogenesis is one of the important mechanisms of tumorigenesis.

As such, angiogenesis-related diseases exhibit serious conditions, and various therapeutic agents have been developed to treat these diseases. However, these therapeutic agents may not exhibit proper therapeutic effects in many cases, because growth factors and signaling mechanisms that mainly contribute to angiogenesis are diverse. In this case, due to repeated administration of an excessive therapeutic dose, resistance to the therapeutic agent is likely to occur and a clear therapeutic effect cannot be expected.

In addition, since a target receptor, which is the target of the therapeutic agent for angiogenesis-related disease, exists in vascular endothelial cells, the binding between the target-specific targeting substance and the receptor is dissociated within a short time due to blood pressure, blood flow, vascular permeability, etc., and thus the therapeutic agent may be released. Therefore, for a long-term therapeutic effect, the therapeutic agent is repeatedly administered at a high dose, causing various side effects.

Meanwhile, targeted cancer therapy, which has recently attracted attention, is a therapeutic method that targets a tumor-specific biomarker, unlike existing therapeutic methods, and specific examples thereof include targeted anticancer drug therapy, therapeutic radioisotope therapy, photodynamic therapy, and the like. Since pharmaceutical drugs used for targeted tumor therapy are delivered by targeting tumor-specific biomarkers, they induce tumor cell death while minimizing damage to normal cells. In order for a drug used for such targeted cancer therapy to exhibit an effective anticancer effect, the drug must have high selectivity and binding affinity to tumor cells, and also be effectively delivered to tumor cells through cellular internalization.

In order to enhance the cellular internalization of drugs, a ligand including a multimeric compound capable of binding to multiple target receptors simultaneously has been developed (Wu Z. et al., J Nucl Med 2007; 48:1536-1544). However, this ligand has limitations in endocytosis *in vivo* due to the positional specificities (blood pressure, blood flow, vascular permeability, etc.) of vascular endothelial cells, and has a disadvantage in that the effect of internalizing the drug into cells is low.

Accordingly, the present inventors have paid attention to the fact that, in the case of conventional drug conjugates used for the diagnosis or treatment of various diseases such as angiogenesis-related diseases, the internalization efficiency of the drug is very low due to limited endocytosis *in vivo,* and expected that, if the cellular internalization of a drug conjugate by endocytosis is enhanced, the sensitivity and accuracy of diagnosis may be significantly improved, and the targeted therapeutic efficacy of the drug may be significantly improved. To this end, the present inventors have made extensive efforts to develop a novel drug conjugate and drug delivery platform, and as a result, have found that, when a binding group is additionally introduced to a drug conjugate in which a targeting substance and a drug are linked together, an interaction between the binding group and another binding group introduced to another drug conjugate may be induced, each of the drug conjugates may bind to target cells, and cross-linking between the drug conjugates *in vivo* may be induced to form a cluster, thus activating endocytosis and significantly increasing the cellular internalization of the drug included in the drug conjugates. Based on this finding, the present invention has been completed.

### [Prior Art Documents]

### [Patent Documents]

(Patent Document 1) PCT/KR2011/003801

[Non-Patent Documents]
(Non-Patent Document 1) Conner S.D. et al., Nature 2003; 422:37-44;
(Non-Patent Document 2) Marsh M. et al., Cell 2006; 124: 729-40;
(Non-Patent Document 3) Smith A.E. et al., Science 2004; 304:237-42;
(Non-Patent Document 4) Wu Z. et al., J Nucl Med 2007; 48:1536-1544.

### Summary of the Invention

An object of the present invention is to provide a novel drug conjugate having improved drug delivery efficiency due to significantly increased cellular internalization.

To achieve the above object, the present invention provides a unit drug conjugate wherein a binding group capable of binding to another unit drug conjugate is additionally linked to a drug conjugate in which a target substance, which specifically binds to target cells, and a drug, are linked together.

The present invention also provides a drug conjugate comprising: the unit drug conjugate (a first unit drug conjugate); and another drug conjugate (a second unit drug conjugate) capable of binding to the unit drug conjugate by a binding group.

The present invention also provides a pharmaceutical composition for treating various diseases, preferably a pharmaceutical composition for treating angiogenesis-related diseases, the pharmaceutical composition comprising the drug conjugate.

The present invention also provides a composition for diagnosing various diseases, preferably a composition for diagnosing angiogenesis-related diseases, the composition comprising the drug conjugate.

The present invention also provides a pharmaceutical composition for diagnosing and treating various diseases, preferably a pharmaceutical composition for diagnosing and treating angiogenesis-related diseases, the pharmaceutical composition comprising the drug conjugate.

The present invention also provides a method for treating various diseases comprising a step of administering the drug conjugate.

The present invention also provides a method for diagnosing various diseases comprising a step of using the drug conjugate.

The present invention also provides the use of the drug conjugate for treating various diseases.

The present invention also provides the use of the drug conjugate for diagnosing various diseases.

The present invention also provides the use of the drug conjugate in the manufacture of a medicament for the treatment of various diseases.

### Brief Description of Drawings

FIG. 1A is a schematic view showing the structure of a unit drug conjugate according to the present invention.
FIG. 1B is a schematic view showing the principle of operation of the present invention.
FIG. 2 shows the results of confirming the cross-linking between unit drug conjugates in a cell-like environment according to an example of the present invention.
FIG. 3A depicts fluorescence images showing the results of confirming the effect of promoting endocytosis by the formation of cross-linking between conjugates according to an example of the present invention.
FIG. 3B shows the results of quantifying the fluorescence intensities of fluorescence images showing the results of confirming the effect of promoting endocytosis by the formation of cross-linking between conjugates according to an example of the present invention.
FIG. 3C shows the results of Z-stack analysis of fluorescence images showing the results of confirming the effect of promoting endocytosis by the formation of cross-linking between conjugates according to an example of the present invention.
FIG. 4 shows the results of an *ex vivo* biodistribution experiment on drug conjugates according to an example of the present invention.
FIG. 5 shows the results of examining the difference in retention in a tumor depending on the sequential doses of unit drug conjugates according to an example of the present invention.
FIG. 6 shows the results of examining the effect of enhancing retention in a tumor by sequential administration of unit drug conjugates according to an example of the present invention.

### Detailed Description and Preferred Embodiments of the Invention

Unless otherwise defined, all technical and scientific terms used in the present specification have the same meanings as commonly understood by those skilled in the art to which the present disclosure pertains. In general, the nomenclature used in the present specification is well known and commonly used in the art.

In the present invention, it was found that, when *in vivo* binding of binding groups between unit drug conjugates in which a targeting substance and a drug are linked together was induced, the drug conjugates (complexes) bound to target cells were clustered by cross-linking, and then endocytosis of the drug conjugates was promoted and cellular internalization thereof was enhanced, and thus the intracellular concentration and retention of the drug were enhanced.

Therefore, in one aspect, the present invention is directed to a unit drug conjugate wherein a binding group capable of binding to another drug conjugate is additionally linked to a drug conjugate in which a targeting substance, which binds specifically to target cells, and a drug, are linked together.

In the present invention, the targeting substance may specifically bind to a target that is specifically expressed or overexpressed in target cells, and the drug may be a therapeutic or diagnostic drug, without being limited thereto.

In the present invention, the targeting substance and the drug may be linked together by a linker, but the targeting substance and the drug may also be linked together directly without a linker.

In the present invention, the binding group may be further linked to the linker, but may also be linked to other site of the drug conjugate. In the present invention, the binding group may be one or more.

In another aspect, the present invention is directed to a drug conjugate comprising: the unit drug conjugate (a first unit drug conjugate); and another drug conjugate (a second unit drug conjugate) capable of binding to the unit drug conjugate by a binding group.

In the present invention, when the binding group included in the first unit drug conjugate is named a first bonding group and the binding group included in the second unit drug conjugate is named a second bonding group, the first unit drug conjugate and the second unit drug conjugate may be characterized in that the first binding group and the second binding group bind to each other.

In the present invention, the targeting substance introduced to the first unit drug conjugate and the targeting substance introduced to the second unit drug conjugate may be the same as or different from each other, but all of the targeting substances may bind specifically to targets that are specifically expressed or overexpressed in the same target cells.

In the present invention, the drug introduced to the first unit drug conjugate and the drug introduced to the second unit drug conjugate may be the same as or different from each other.

The present invention may also provide a multi-drug conjugate comprising a plurality of unit drug conjugates that may be bound together by one or more binding group pairs.

The present invention also provides a unit drug conjugate pair comprising: the unit drug conjugate (a first unit drug conjugate); and another drug conjugate (a second unit drug conjugate) having a second binding group capable of binding to a first binding group of the first unit drug conjugate.

The present invention also provides a plurality of unit drug conjugates comprising: the unit drug conjugate (a first unit drug conjugate); and another drug conjugate (a second unit drug conjugate) having a second binding group capable of binding to a first binding group of the first unit drug conjugate.

The second unit drug conjugate may be a unit drug conjugate wherein the second binding group capable of binding to the first binding group of the first unit drug conjugate is additionally linked to a drug conjugate in which a second targeting substance, which binds specifically to a target that is specifically expressed or overexpressed in target cells, and a therapeutic or diagnostic drug, are linked together.

The present invention is also directed to a second unit drug conjugate wherein a second binding group capable of binding to a first binding group of a first unit drug conjugate is additionally linked to a drug conjugate in which a second targeting substance, which binds specifically to a target that is specifically expressed or overexpressed in target cells, and a therapeutic or diagnostic drug, are linked together.

Furthermore, the present invention is directed to a drug conjugate formed by binding between the first binding group of the first unit drug conjugate and the second binding group of the second unit drug conjugate.

When the first unit drug conjugate and/or the second unit drug conjugate according to the present invention are/is used, it is possible to rapidly target a target cell, for example, a cancer cell. That is, when an existing anticancer drug has low tumor uptake pharmacokinetics, it inhibits normal cells, and the efficacy thereof is reduced due to rapid *in vivo* metabolism thereof, and thus high-dose administration of the drug is necessary. However, when the first unit drug conjugate and/or the second unit drug conjugate according to the present invention are/is used, there is an advantage in that the unit drug conjugate(s) are stable in blood and may rapidly and selectively target the cell membrane protein of a tumor in an amount that has little to no toxicity.

Furthermore, when the first unit drug conjugate and/or the second unit drug conjugate according to the present invention are/is used, there is an advantage in that tumor cellular internalization of the drug may be enhanced. That is, cancer cellular internalization of an existing anticancer drug relies only on the natural intracellular uptake mechanism, but when the first unit drug conjugate and/or the second unit drug conjugate according to the present invention are/is used, crosslinking between the first unit drug conjugate and/or second unit drug conjugate rapidly and selectively bound to a target cell (e.g., the target site of cancer) occurs by *in vivo* intermolecular interactions and a plurality of complexes (complexes between the membrane protein and the targeting substance) formed by the crosslinking are grouped to form a cluster. This cluster accelerates endocytosis and consequently enhances cellular internalization of the drug. This shows a surprising effect of artificially enhancing the known natural cellular internalization.

In the present invention, as the targeting substance (first targeting substance) introduced to the first unit drug conjugate and the targeting substance (second targeting substance) introduced to the second unit drug conjugate, any substances may be used without limitation, as long as they are substances capable of binding specifically to a target that is specifically expressed or overexpressed in target cells.

In the present invention, "specifically expressed in target cells" means that the target is not expressed in normal cells but is specifically expressed only in target cells, and "overexpressed in target cells" means that the target is abnormally highly expressed in target cells compared to normal cells.

The first targeting substance and the second targeting substance may target the same target or different targets, and may be different substances even when they target the same target.

For example, when the first targeting substance and the second targeting substance are used, which target the epidermal growth factor (EGF) specifically expressed on the surfaces of cancer cells, both the first targeting substance and the second targeting substance may be antibodies against the EGF receptor, or the first targeting substance may be an antibody against the EGF receptor, and the second targeting substance may be the EGF which is a ligand for the EGF receptor.

Furthermore, even when the first targeting substance and the second targeting substance are both antibodies (preferably monoclonal antibodies) to the EGF receptor, they may be antibodies having different CDRs (complementarity-determining regions) or variable regions.

The substance capable of specifically binding to a target specifically expressed in target cells may be selected from among antibodies, aptamers, peptides such as ligands, carbohydrates, and small molecule compounds, without being limited thereto.

As used herein, the term "antibody" includes not only the whole antibody form, but also an antigen-binding fragment of the antibody molecule.

The whole antibody has a structure having two full-length light chains and two full-length heavy chains, and the light chains are linked with the heavy chains via disulfide bonds, respectively. Each heavy chain constant region has gamma (γ), mu (m), alpha (α), delta (δ), and epsilon (ε) types, and gamma1 (γ1), gamma2 (y2), gamma3 (γ3), gamma4 (γ4), alpha1 (α1), and alpha2 (α2) subclasses. Each light chain constant region has kappa (κ) and lambda (λ) types.

As used herein, the term "antigen binding fragment of the antibody" or "antibody fragment" refers to a fragment that retains an antigen binding function, and includes Fab, F(ab'), F(ab')2, and Fv. Among the antibody fragments, Fab has a structure having heavy chain and light chain variable regions, a light chain constant region, and a first heavy chain constant region (CH1), and Fab has one antigen binding site. Fab' differs from Fab in that it has a hinge region including one or more cysteine residues at the C-terminus of the heavy chain CH1 domain. F(ab')2 antibody is generated through a disulfide bond formed between the cysteine residues in the hinge regions of Fab' fragments. Fv is a minimal antibody segment having only a heavy chain variable domain and a light chain variable domain. A two-chain Fv has a structure in which a heavy chain variable region and a light chain variable region are linked together through a noncovalent bond, and a single-chain Fv (scFv) generally comprises a heavy chain variable region and a light chain variable region covalently linked to each other via a peptide linker or directly linked at the C-terminus, thereby forming a dimeric structure as in the two-chain Fv. These antibody fragments may be obtained using proteases (for example, the whole antibody may be restriction-digested with papain to obtain Fab fragments, and may be digested with pepsin to obtain F(ab')2 fragments), and may also be generated by a genetic recombinant technique.

An "Fv" fragment is an antibody fragment containing the complete antibody recognition and binding sites. Such a region includes a dimer, e.g., scFv that consists of one heavy-chain variable domain and one light-chain variable domain substantially tightly covalently linked to each other.

The "Fab" fragment contains a variable domain and a constant domain of the light chain and a variable domain and a first constant domain (CH1) of the heavy chain. The F(ab')2 antibody fragment generally includes a pair of Fab fragments covalently linked via a hinge cysteine located therebetween near the carboxyl end thereof.

The "single-chain Fv" or "scFv" antibody fragment includes VH and VL domains of the antibody, wherein these domains are present in a single polypeptide chain. The Fv polypeptide may further include a polypeptide linker between the VH domain and the VL domain in order for the scFv to form a desired structure for antigen binding.

In the present invention, the targeting substance of the drug conjugate refers to a substance capable of binding to a target that is specifically expressed or overexpressed in target cells, and examples thereof include, but are not limited to, an RGD (arginine (R)-glycine (G)-aspartic acid (D)) peptide that specifically binds to integrin αvβ3, a glutamate-urea-lysine (GUL) motif that binds to prostate specific membrane antigen (PSMA), EGF that binds to EGF receptor, VEGF-A or VEGF-B that binds to vascular endothelial growth factor (VEGF) receptor, and the like.

As described above, the target that is specifically expressed or overexpressed in target cells refers to a protein that is specifically expressed in a particular disease or overexpressed compared to that in normal cells, and examples thereof include, but are not limited to, receptors or tumor-specific antigens, and the like.

Examples of the target that is specifically expressed in target cells include, but are not limited to, integrins such as integrin αvβ3, prostate specific membrane antigen (PSMA), CD3, CD4, CD6, CD11a, CD19, CD20, CD22, CD30, CD33, CD38, CD40, CD52, CD62, CD79b, CD80, CGRP, OX-40, CTLA4, 4-1BB, PD-1, EGF receptor, TNF receptors such as TNF (tumor necrosis factor)-α, Fc receptor, folate receptor, GD2, HER2, Her2/neu, HER3, HER4, VEGF receptor, interferon receptor, IgE receptor, IGF-1 receptor, interleukin-2 receptor, interleukin-5 receptor, interleukin-6 receptor, interleukin-17 receptor A, interleukin-31 receptor, interleukin-36 receptor, B7-H3, and CCR4.

For example, an RGD (arginine (R)-glycine (G)-aspartic acid (D)) peptide that specifically binds to integrin αvβ3, a glutamate-urea-lysine (GUL) motif targeting prostate specific membrane antigen (PSMA), an antibody, an aptamer, a small molecule compound, or a tumor-targeting peptide may be used, without being limited thereto.

The RGD peptide that is used in one embodiment of the present invention is a targeting substance that specifically binds to integrin αvβ3, which is a membrane protein involved in tumor neovascularization, and it is based on three amino acids (arginine-glycine-aspartic acid) and may be used as a targeting substance for the diagnosis or treatment of tumors. Meanwhile, the glutamate-urea-lysine motif that may be used in another embodiment may be a targeting substance that targets PSMA, which is known as a biomarker of prostate cancer, without being limited thereto.

In the present invention, the target cells are cells that are a target for diagnosis or treatment of diseases, preferably angiogenesis-related diseases, or cells that are a target for diagnosis or treatment of prostate cancer, and examples thereof include tumor cells, atherosclerosis-causing cells, myocardial infarction-causing cells, etc.

In the present invention, the drugs included in the first unit drug conjugate and the second unit drug conjugate may be the same drug or different drugs.

The drug is a concept including both a diagnostic drug and a therapeutic drug. The diagnostic drug may be a fluorescent dye or a diagnostic gamma-ray/positron emitting radioisotope, without being limited thereto. The therapeutic drug may be selected from the group consisting of photosensitizers that are used for photodynamic therapy, isotope boron (10B)-containing molecules that are used for boron neutron capture therapy, alpha/beta radiation emitting therapeutic radioisotopes that are used for nuclear medicine therapy, and anticancer drugs that are used for anticancer chemotherapy, without being limited thereto. The drug may be a small molecule compound, a synthetic pharmaceutical drug, a peptide, a protein or an antibody, without being limited thereto.

For example, the drug may be at least one selected from the group consisting of maytansinoid, auristatin, aminopterin, actinomycin, bleomycin, talisomycin, camptothecin, N8-acetyl spermidine, 1-(2-chloroethyl)-1,2-dimethylsulfonyl hydrazide, esperamycin, etoposide, 6-mercaptopurine, dolastatin, tricotecene, calicheamycin, taxol, taxane, paclitaxel, docetaxel, methotrexate, vincristine, vinblastine, doxorubicin, melphalan, mitomycin A, mitomycin C, chlorambucil, duocarmycin, L-asparaginase, mercaptopurine, thioguanine, hydroxyurea, cytarabine, cyclophosphamide, ifosfamide, nitrosourea, cisplatin, carboplatin, mitomycin, dacarbazine, procarbazine, topotecan, nitrogen mustard, cytoxan, etoposide, 5-fluorouracil, bischloroethylnitrosourea (BCNU), irinotecan, camptothecin, bleomycin, idarubicin, daunorubicin, dactinomycin, plicamycin, mitoxantrone, asparaginase, vinorelbine, chlorambucil, melphalan, carmustine, lomustine, busulfan, treosulfan, decarbazine, etoposide, teniposide, topotecan, 9-aminocamptothecin, crisnatol, mitomycin C, trimetrexate, mycophenolic acid, tiazofurin, ribavirin, 5-ethynyl-1-beta-dribofuranosylimidazole-4-carboxamide (EICAR), hydroxyurea, deferoxamine, floxuridine, doxifluridine, raltitrexed, cytarabine (ara C), cytosine arabinoside, fludarabine, tamoxifen, raloxifene, megestrol, goserelin, leuprolide acetate, flutamide, bicalutamide, EB1089, CB1093, KH1060, verteporfin, phthalocyanine, photosensitizer Pe4, demethoxy-hypocrellin A, interferon-α, interferon-γ, tumor necrosis factor, gemcitabine, velcade, revamid, thalamid, lovastatin, 1-methyl-4-phenylpyridinium ion, staurosporine, actinomycin D, dactinomycin, bleomycin A2, bleomycinB2, peplomycin, epirubicin, pirarubicin, zorubicin, mitoxantrone, verapamil, thapsigargin, temozolomide, nuclease, toxins derived from bacteria or animals/plants, a radioisotope, and fluorescent dyes, without being limited thereto.

Illustratively, the radioisotope may be an isotope that emits gamma rays or positrons to provide diagnostic images, or emits beta or alpha rays to provide therapeutic efficacy, and may be, for example, ¹¹C, ¹⁸F, ⁹⁹mTc, ¹⁸⁸Re, ^{125/123/124/131}I, ⁸⁹Zr, ^{64/67}Cu, ⁶⁸Ga, ¹⁷⁷Lu, ⁹⁰Y, ²²⁵Ac, or ²¹¹At, without being limited thereto.

Illustratively, the fluorescent dye may be fluorescein isothiocyanate (FITC), tetramethylrhodamine (TRITC), alexa fluor series or cyanine (Cy) series fluorescent dye, without being limited thereto.

It is preferable that the drugs included in the first unit drug conjugate and the second unit drug conjugate according to the present invention are different from each other. When the drugs included in the first unit drug conjugate and the second unit drug conjugate are different from each other as described above, there is an advantage in that combination therapy may be performed in a customized manner through the loading and delivery of multiple drugs. That is, the resistance of cancer to an existing anticancer agent shortens the use cycle of the anticancer agent and eventually causes the patient to stop treatment; however, by introducing various drugs to the first unit drug conjugate and the second unit drug conjugate for the purpose of diagnosing and treating cancers, customized therapy and combination therapy corresponding to the resistance are possible.

In the present invention, the first binding group and the second binding group may be used without limitation as long as they are binding groups that may be bound *in vivo.* The binding groups may be binding groups for binding by click reaction, binding groups for binding by host-guest chemical interaction, or avidin-biotin binding groups, without being limited thereto.

In the present invention, the binding may be binding by click reaction, binding by host-guest interaction, or avidin-biotin binding, without being limited thereto. It will be apparent to those skilled in the art that any binding may be used without limitation, as long as *in vivo* crosslinking may occur to induce the interaction between two or more unit drug conjugates.

Specifically, the binding groups include binding groups that use a click reaction. Among various types of click reactions, the copper catalyst-free click reaction is known as a reaction in which binding occurs within a short time even in an aqueous environment such as an *in vivo* environment (Jewett J.C. et al. Chem. Soc. Rev. 2010; 39:1272-1279). In some embodiments, the binding groups for binding by click reaction may be azide-azadibenzocyclooctyne (ADIBO) binding groups, trans cyclooctene (TCO)-tetrazine binding groups, or alkyne-cyclopentadienone binding groups, without being limited thereto.

In the present invention, the host-guest interaction is noncovalent binding between host and guest compounds, which shows high binding strength (Yu G. et al., Theranostics. 2019; 9:3047-3074). In particular, the host-guest interaction is applicable to the present invention because it shows a high binding strength between the compounds even in an environment similar to an *in vivo* environment. In one embodiment, the binding groups for binding by host-guest interaction may be cucurbituril-adamantane or cyclodextrin-amino acid binding groups, without being limited thereto.

It is also possible for the binding groups to use an avidin-biotin interaction. The avidin-biotin interaction is selective binding and is one of the strong non-covalent interactions that exist in nature, and has the advantage of having a stronger binding strength than antibody-antigen binding above all else (Jain A. et al. J Control Release. 2017; 245: 27-40).

In the present invention, the first targeting substance and drug included in the first unit drug conjugate and/or the second targeting substance and drug included in the second unit drug conjugate may be linked together by a linker or linked directly without a linker.

In addition, the first targeting substance and first binding group included in the first unit drug conjugate and/or the second targeting substance and second binding group included in the second unit drug conjugate may be linked together by a linker or linked directly without a linker.

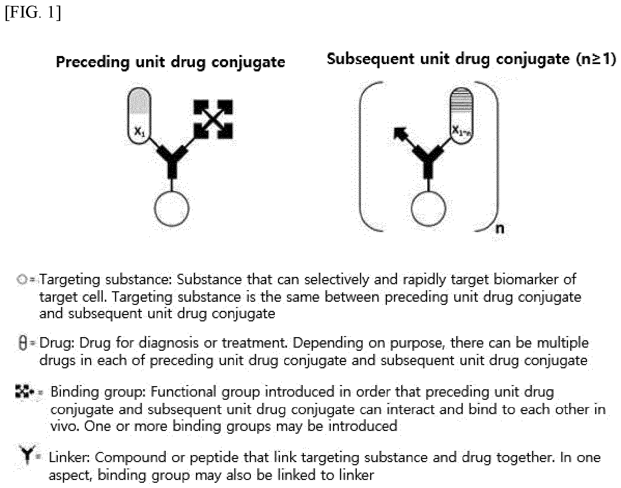

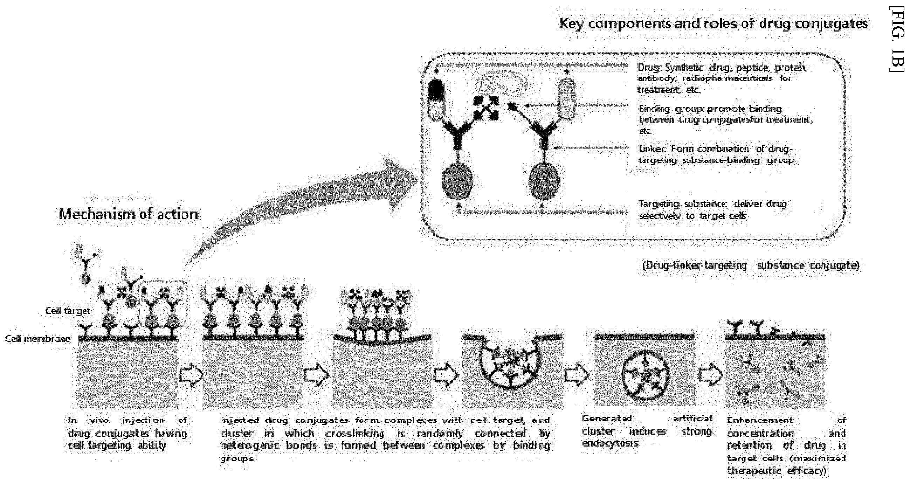

In a preferred embodiment, the first targeting substance, drug, and first binding group included in the first unit drug conjugate are linked together by a linker (linking group) having three or more functional groups as shown in FIG. 1A, without being limited thereto. The first targeting substance, drug, and first binding group may be linked together by a linker or may be directly linked together.

In the present invention, the linker may be an amino acid, hydrocarbon or PEG chain, without being limited thereto. That is, as the linker, any substance may be used without limitation, as long as it is a substance known in the art having an atomic or molecular group and other functional groups suitable for linking the targeting substance and the drug together and additionally linking the binding group thereto.

In still another aspect, the present invention is directed to a drug conjugate comprising: the unit drug conjugate (first unit drug conjugate); and another drug conjugate (second unit drug conjugate) capable of binding to the unit drug conjugate via binding groups (a first binding group and a second binding group).

In the present invention, the binding may be binding by click reaction, binding by host-guest interaction, or avidin-biotin binding, without being limited thereto.

In the present invention, the binding by click reaction may be azide-ADIBO binding, TCO-tetrazine binding, or alkyne-cyclopentadienone binding, without being limited thereto.

In the present invention, the binding by host-guest interaction may be cucurbituril-adamantane binding, or cyclodextrin-amino acid binding, without being limited thereto.

In yet another aspect, the present invention is directed to a pharmaceutical composition for treating angiogenesis-related diseases containing the unit drug conjugate.

The pharmaceutical composition may comprise two or more different unit drug conjugates. The term "different two or more unit drug conjugates" may mean that the binding groups that function to allow interaction between the drug conjugates constituting each unit drug conjugate are different between two or more unit drug conjugates. Also, it may mean that the drugs bound to a plurality of unit drug conjugates comprising two or more unit drug conjugates are different from each other.

In the present invention, the first unit drug conjugate and the second unit drug conjugate may be administered simultaneously, but preferably, they may be administered sequentially. In a preferred embodiment of the present invention, the first unit drug conjugate is administered prior to the second unit drug conjugate, and after the first unit drug conjugate reaches and binds to target cells, the second unit drug conjugate is administered.

In one embodiment, the second unit drug conjugate is administered 1 minute to 600 minutes, preferably 5 minutes to 480 minutes, more preferably 10 minutes to 300 minutes, most preferably 30 minutes to 240 minutes after administration of the first unit drug conjugate.

The second unit drug conjugate may be used in the same amount as the first unit drug conjugate. Meanwhile, one or more unit drug conjugates may be used per first unit drug conjugate, and for example, 1 to 10 types, preferably 1 to 5 types, of unit drug conjugates may be used per first unit drug conjugate, without being limited thereto.

In the present invention, the angiogenesis-related disease may be selected from the group consisting of benign tumor, malignant tumor (cancer), diabetic retinopathy, age-related macular degeneration, rheumatoid arthritis, endometriosis, psoriasis, chronic inflammation, coronary artery disease, atherosclerosis, stroke, ulcer, and myocardial infarction, without being limited thereto.

In still yet another aspect, the present invention is directed to a composition for diagnosing angiogenesis-related disease comprising the unit drug conjugate.

In the present invention, the angiogenesis-related disease may be selected from the group consisting of benign tumor, malignant tumor (cancer), diabetic retinopathy, age-related macular degeneration, rheumatoid arthritis, endometriosis, psoriasis, chronic inflammation, coronary artery disease, atherosclerosis, stroke, ulcer, and myocardial infarction, without being limited thereto.

In a further aspect, the present invention is directed to a composition for diagnosing and treating angiogenesis-related diseases comprising the unit drug conjugate so that diagnosis and treatment may be performed simultaneously.

In the present invention, the first unit drug conjugate may be administered prior to the second unit drug conjugate, the drug included in the first unit drug conjugate may be a diagnostic drug, and the drug included in the second unit drug conjugate may be a therapeutic drug.

In the present invention, the first unit drug conjugate may be administered prior to the second unit drug conjugate, the drug included in the first unit drug conjugate may be a therapeutic drug, and the drug included in the second unit drug conjugate may be a diagnostic drug.

In the present invention, the angiogenesis-related disease may be selected from the group consisting of benign tumor, malignant tumor (cancer), diabetic retinopathy, age-related macular degeneration, rheumatoid arthritis, endometriosis, psoriasis, chronic inflammation, coronary artery disease, atherosclerosis, stroke, ulcer, and myocardial infarction, without being limited thereto.

In the present invention, the pharmaceutical composition may be prepared into any one formulation selected from the group consisting of injections, oral formulations, patches, solutions, capsules, granules, tablets, powders, sprays, ointments, gels, formulations for mucosal administration, and suppositories, without being limited thereto. These formulations may be prepared by a conventional method used for formulation in the art or by a method disclosed in Remington's Pharmaceutical Science (latest edition), Mack Publishing Company, Easton PA, and may be prepared in various forms depending on each disease or components. However, the above description is exemplary, and formulations to which the present invention is applicable are not limited to the above description.

In the present invention, the pharmaceutical composition may further contain acceptable excipients, and the adjuvant may be, for example, a carrier. A pharmaceutically acceptable carrier that may be used is one or more of saline, sterile water, Ringer's solution, buffered saline, dextrose solution, maltodextrin solution, glycerol, ethanol, and mixtures thereof. If necessary, other conventional additives such as antioxidants, buffers, and bacteriostats may be added. In addition, the pharmaceutical composition may be formulated into injectable formulations, such as aqueous solutions, suspensions or emulsions, pills, capsules, granules, or tablets by additionally adding diluents, dispersants, surfactants, binders and lubricants. However, the above description is exemplary, and the adjuvants or carriers usable in the present invention are not limited to the above description.

The composition of the present invention may be administered parenterally (e.g., intravenously, subcutaneously, orally, intraperitoneally or topically) according to a desired method, and the dosage thereof may vary depending on the patient's body weight, age, sex, health status, and diet, the duration of administration, the mode of administration, excretion rate, and the severity of the disease. In this case, the dosage regimen and dosage will vary depending on the age, weight and response of an individual patient. An appropriate dosage regimen and dosage should be determined by one of ordinary skill in the art taking these factors into consideration.

The constitution of the unit drug conjugate is divided into a targeting substance, a binding group, a drug, and a linker, as shown in FIG. 1A, and the role of each component is as summarized below.

### Targeting substance

The targeting substance refers to a substance capable of binding to a target that is specifically expressed or overexpressed in target cells. Illustratively, the targeting substance refers to a ligand that specifically binds to an integrin receptor involved in tumor angiogenesis, a ligand that binds to a receptor involved in PSMA, a ligand that binds to EGF receptor, a ligand that binds to vascular endothelial growth factor (VEGF) receptor, and the like, without being limited thereto.

### Binding group

The binding groups are functional groups that induce *in vivo* crosslinking between different unit drug conjugates, and may spontaneously bind to each other covalently or non-covalently bond when positioned close to each other. Examples of the binding include, but are not limited to, binding by click reaction, binding by host-guest interaction, or avidin-biotin binding.

The binding groups that are used for binding by click reaction may be azide-ADIBO, TCO-tetrazine, or alkyne-cyclopentadienone, without being limited thereto.

The binding groups that are used for binding by host-guest interaction may be cucurbituril-adamatane, or cyclodextrin-amino acid, without being limited thereto.

The avidin-biotin binding groups may be avidin-biotin, without being limited thereto. Methods in which a plurality of unit drug conjugates bind together via binding groups are very diverse, and some embodiments thereof will now be described in detail.

One or plural second unit drug conjugates may bind to the first unit drug conjugate according to the present invention.

When one second unit drug conjugate binds to the first unit drug conjugate, binding by click reaction between the binding groups, binding between the binding groups by host-guest interaction, or avidin-biotin binding between the binding groups may be used for *in vivo* crosslinking. Illustratively, when the binding groups for binding between unit drug conjugates are expressed as "A" and "a", respectively, the unit drug conjugates may comprise one "A" and one "a", respectively. When a plurality of second unit drug conjugates bind to the first unit drug conjugate, the binding group structure of the first unit drug conjugate may be more diverse than the binding group structure of one second unit drug conjugate. Specifically, in the binding method selected for *in vivo* crosslinking, the binding groups introduced to the unit drug conjugates may be expressed as "A" and "a", respectively. In this case, the first unit drug conjugate may comprise one or more "A" as a binding group, and this unit drug conjugate may bind to a plurality of second unit drug conjugates comprising "a". In this case, a plurality of "A" included in the unit drug conjugate may be linked to the unit drug conjugate in various forms, and specifically, as shown below, the plurality of "A" may be linked in a linear form, a cyclic form, a branched form, or the like, without being limited thereto. In this case, "A" means a binding group that may interact as described in the above-described binding group, and specific examples thereof include azide-ADIBO, TCO-tetrazine, alkyne-cyclopentadienone, cucurbituril-adamantane, cyclodextrin-amino acid, avidin-biotin, etc. The number of binding groups may be 1 to 30. As shown in FIG. 1A, "Y" is a linker, and " 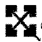 " is a binding group. The following is an exemplary representation of a linear structure, a cyclic structure, and a branched structure, but the present invention is not limited thereto.

### Linear

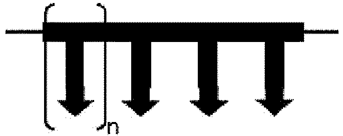

### Cyclic

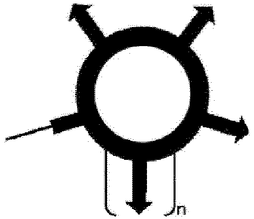

### Branched

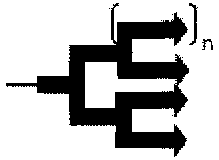

### Drug

In the present invention, the term "drug" is a concept including both a diagnostic drug and a therapeutic drug. The diagnostic drug may be characterized as a fluorescence dye or a diagnostic gamma-ray/positron emitting radioisotope, without being limited thereto. The therapeutic drug may be selected from the group consisting of photosensitizers that are used for photodynamic therapy, isotope boron (¹⁰B)-containing molecules that are used for boron neutron capture therapy, alpha/beta radiation emitting therapeutic radioisotopes that are used for nuclear medicine therapy, and anticancer drugs that are used for anticancer chemotherapy, without being limited thereto.

### Linker

The term "linker" refers to a substance having an atomic group or molecular group and other functional groups for linking a targeting substance and a drug together and additionally linking a binding group thereto. The linker may be an amino acid, hydrocarbon or PEG chain, without being limited thereto. Specifically, the linker may comprise, as a functional group for linking the components together, an amino acid chain having a carboxylic acid and an amine as functional groups, a hydrocarbon chain having an amine (-NH₂), carboxylic acid (-COOH), sulfonic acid (-SH), alcohol (-OH) or halogen group (-Br, Cl, I, etc.) in at least one or more residues, or a PEG chain.

In another aspect, the present invention is directed to a kit for diagnosing angiogenesis-related diseases or a kit for treating angiogenesis-related diseases, the kit containing one or two or more unit drug conjugates described above.

In still another aspect, the present invention is directed to the use of the drug conjugate for treatment of angiogenesis-related diseases.

In yet another aspect, the present invention is directed to the use of the drug conjugate in the manufacture of a medicament for treatment of angiogenesis-related diseases.

In still yet another aspect, the present invention is directed to the use of the drug conjugate for diagnosis of angiogenesis-related diseases.

In a further aspect, the present invention is directed to the use of the drug conjugate in the manufacture of a reagent for diagnosis of angiogenesis-related diseases.

In another further aspect, the present invention is directed to the use of the drug conjugate for treatment and diagnosis of angiogenesis-related diseases.

In still another further aspect, the present invention is directed to the use of the drug conjugate in the manufacture of a medicament for treatment and diagnosis of angiogenesis-related diseases.

The unit drug conjugate according to the present invention may be used in various diagnostic and treatment methods such as fluorescence image-guided surgery, nuclear medicine diagnosis (nuclear imaging), photodynamic therapy (PDT), boron neutron capture therapy (BNCT), radioimmunotherapy, and targeted anticancer chemotherapy.

Therefore, in another aspect, the present invention is directed to a method for treating angiogenesis-related disease comprising steps of:
(a) administering the first unit drug conjugate to a subject in need of treatment for angiogenesis-related disease; and
(b) administering the second unit drug conjugate to the subject.

Therefore, in still another aspect, the present invention is directed to a method for diagnosing angiogenesis-related diseases comprising steps of:
(a) administering the first unit drug conjugate to a subject in need of diagnosis of angiogenesis-related diseases; and
(b) administering the second unit drug conjugate to the subject.

Therefore, in yet another aspect, the present invention is directed to a method for diagnosing and treating angiogenesis-related diseases comprising steps of:
(a) administering the first unit drug conjugate to a subject in need of diagnosis and treatment of angiogenesis-related diseases; and
(b) administering the second unit drug conjugate to the subject.

Taken together, the drug conjugate according to the present invention has the following characteristics.

First, the unit drug conjugate according to the present invention may rapidly target cells on which the unit drug conjugate acts. In general, if a drug does not specifically bind to target cells or the pharmacokinetics of uptake into target cells are slow, problems arise in that the drug inhibits the function of normal cells other than the target cells, and the efficacy of the drug is reduced due to *in vivo* metabolism, resulting in an increase in the dose of the drug. However, since the drug conjugate according to the present invention has introduced thereto a targeting substance that specifically binds to target cells, it may exhibits a high therapeutic effect even when administered at a low dose, thereby preventing toxicity or side effects caused by high-dose administration.

Second, the drug conjugate according to the present invention enhances cellular internalization. While cellular internalization of a conventional tumor cell-targeted therapeutic agent relies only on the natural intracellular uptake mechanism, the unit drug conjugate according to the present invention has introduced thereto a binding group that induces *in vivo* crosslinking, and thus in vivo binding between the unit drug conjugates is induced. In particular, in a sequential administration method in which this unit drug conjugate having a binding group is first administered and then another unit drug conjugate capable of binding to the binding group is administered, the unit drug conjugates bind specifically to the target on target cells because each unit drug conjugates comprises a targeting substance that specifically binds to the target cells. The plurality of unit drug conjugates bound to these target cells are crosslinked *in vivo,* and a cluster is formed by induced *in vivo* crosslinking between a plurality of formed complexes. This cluster accelerates endocytosis and promotes cellular internalization of the drug. Thus, even when the drug is administered at a low dose, it may be effectively delivered to target cells. This is a method of artificially enhancing natural endocytosis. In this method, one or more identical or different binding groups (e.g., binding groups for binding by click reaction, binding groups for binding by host-guest interaction, etc.) may be introduced to the unit drug conjugates, and the unit drug conjugates having different binding groups introduced thereto may interact with and bind to other unit drug conjugates. Accordingly, a cluster may be formed by inducing *in vivo* crosslinking between three or more different unit drug conjugates, and the drugs included in the drug conjugates may also be three or more different drugs.

Finally, according to the present invention, multi-purpose therapy and combination therapy may be in a customized manner. As a plurality of unit drug conjugates may have different binding groups and different drugs and the plurality of different unit drug conjugates may comprise various drugs, they may be very effectively used for multi-purpose therapy and combination therapy. In addition, when one unit drug conjugate of the present invention comprises a diagnostic drug and another unit drug conjugate comprises a therapeutic drug, diagnosis and treatment may be performed simultaneously.

Hereinafter, the present invention will be described in more detail with reference to examples. These examples are only for describing the present invention in more detail, and it will be apparent to those of ordinary skill in the art that the scope of the present invention is not to be construed as being limited by these examples.

### Example 1. Production of drug conjugates

### 1-1. Production of unit drug conjugates

The targeting substances selected in an embodiment of the present invention are RGD peptides that bind to the integrin αᵥβ₃ in tumor angiogenesis, and the structures thereof are as follows.

### Peptide A

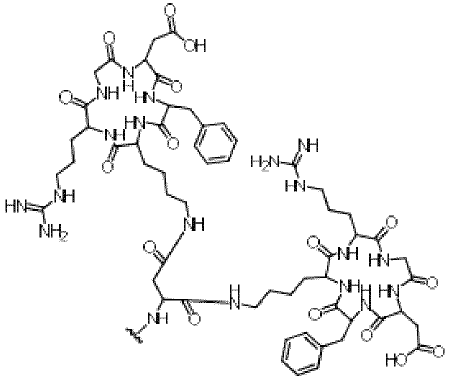

### Peptide B

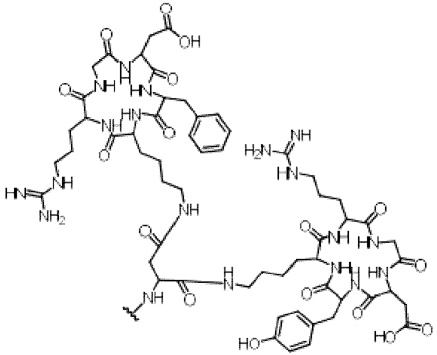

Specifically, peptide A is D-[c(RGDfK)]₂ obtained by linking two cyclicRGDfK with aspartic acid (D), and peptide B is D-c(RGDyK)-c(RGDfK) obtained by linking cyclicRGDfK and cyclicRGDyK containing tyrosine with aspartic acid (D). The targeting substances were synthesized based on BC Lee et al., RSC Advances 2013; 3:782-792.

The unit drug conjugates used in the present invention are as follows. Each unit drug conjugate has a structure in which a targeting substance, a drug and a binding group are linked together via a linker. Various methods of linking components together by a linker are widely known in the art, but in this Example, an amide bond was used to produce each drug conjugate.

### Unit drug conjugate 1

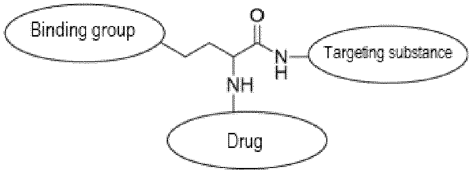

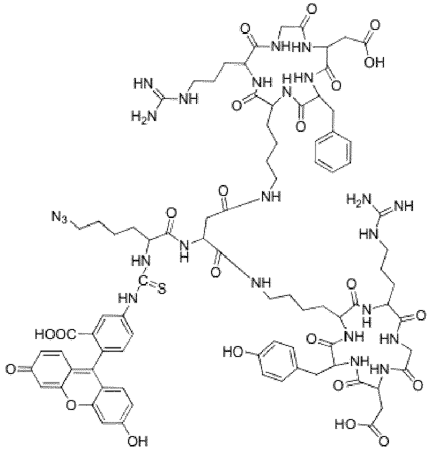

Targeting substance = peptide B, drug = FITC, and binding group = azide

### Unit drug conjugate 1-1

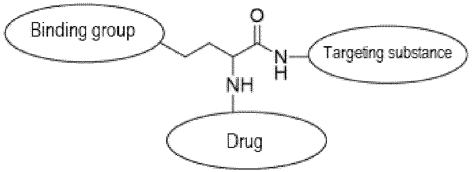

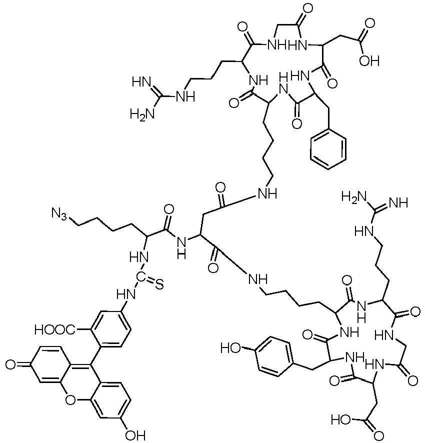

Targeting substance = peptide B, drug = FITC, and binding group = β-CD

### Unit drug conjugate 2

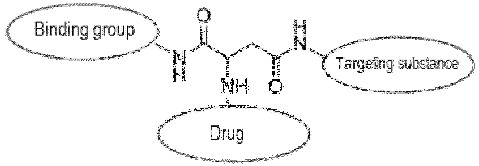

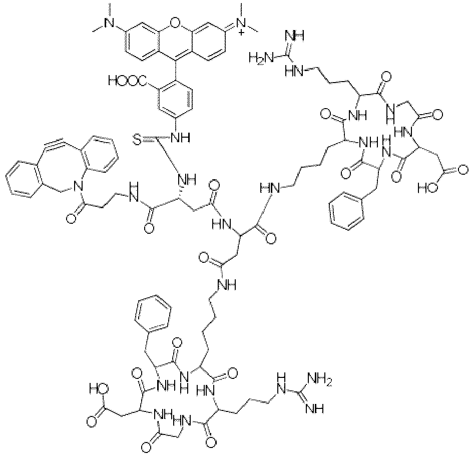

Targeting substance = peptide A, drug = TRITC, and binding group = ADIBO

### Unit drug conjugate 2-1

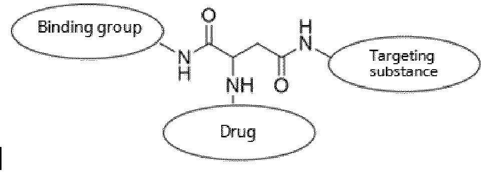

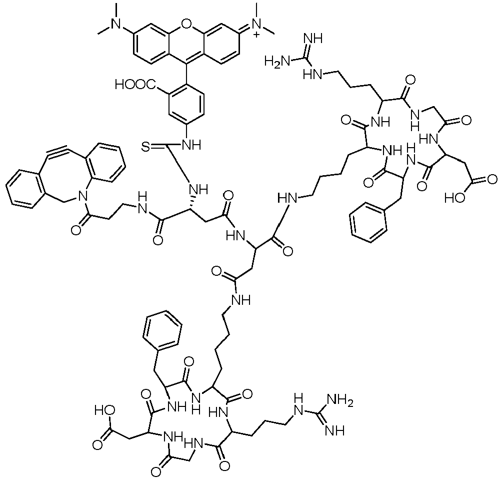

Targeting substance = peptide A, drug = TRITC, and binding group = adamantane

### Unit drug conjugate 3

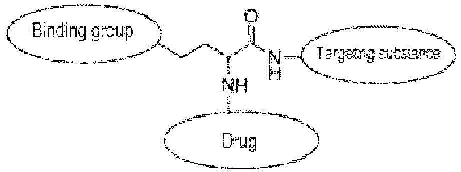

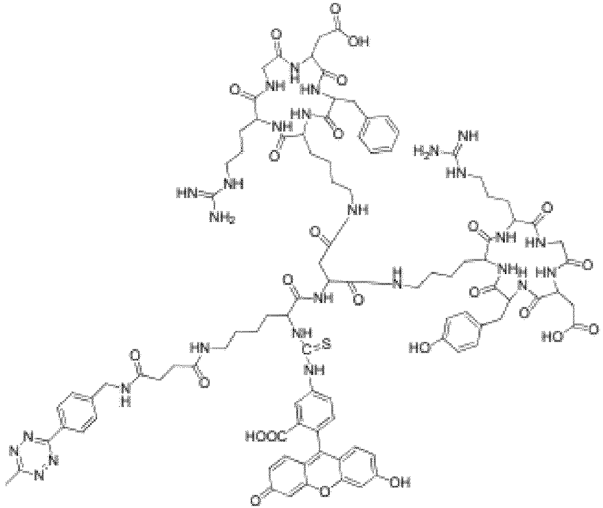

Targeting substance = peptide B, drug = FITC, and binding group = tetrazine

### Unit drug conjugate 4

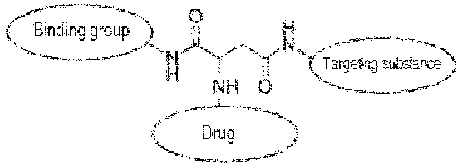

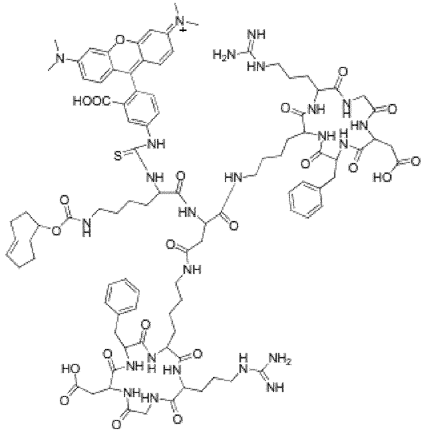

Targeting substance = peptide A, drug = TRITC, and binding group = TCO

### Unit drug conjugate 5

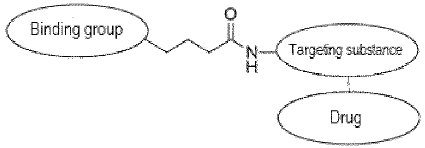

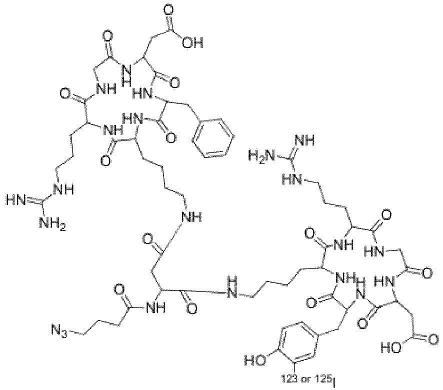

Targeting substance = RGD peptide A, drug = radioisotope iodine (¹²³I or ¹²⁵I), and binding group = azide

### Unit drug conjugate 6

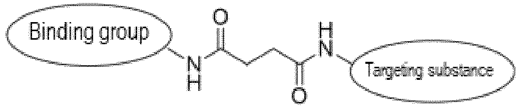

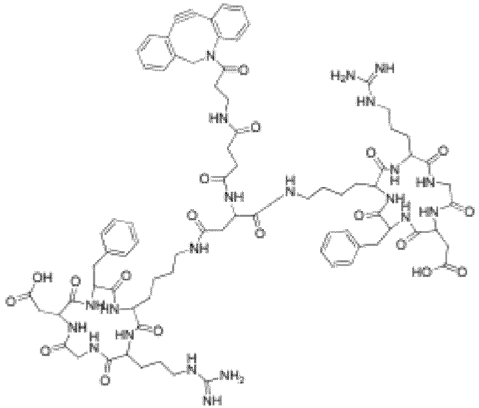

Targeting substance = RGD peptide B, and binding group = ADIBO

### Unit drug conjugate 7

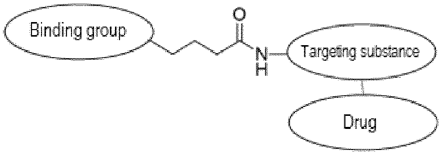

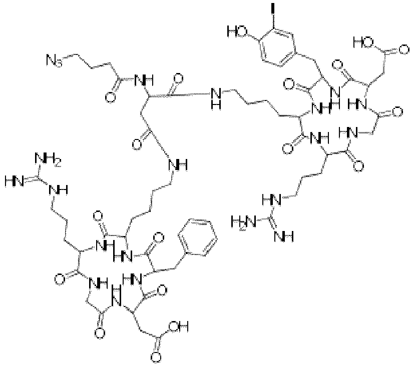

Targeting substance = RGD peptide A, drug = iodine, and binding group = azide

### Unit drug conjugate 8

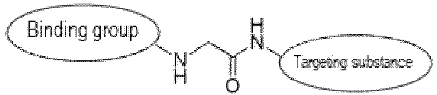

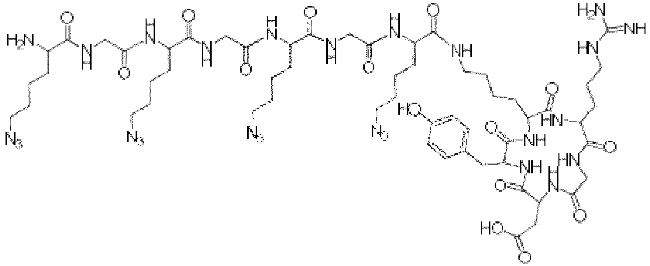

Targeting substance = cyclicRGDyK, and binding group =

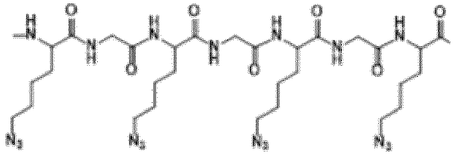

### Unit drug conjugate 9

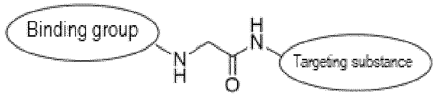

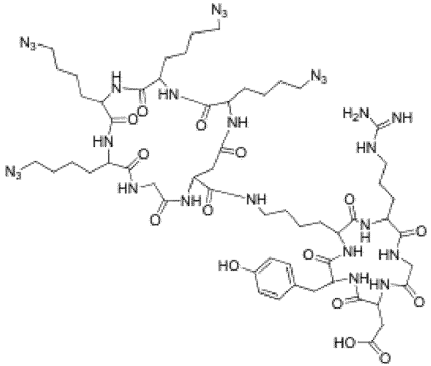

Targeting substance = cyclicRGDyK, and binding group =

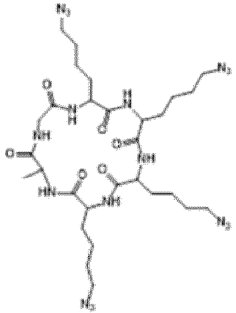

### Unit drug conjugate (9 a-b)

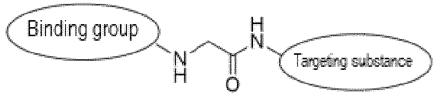

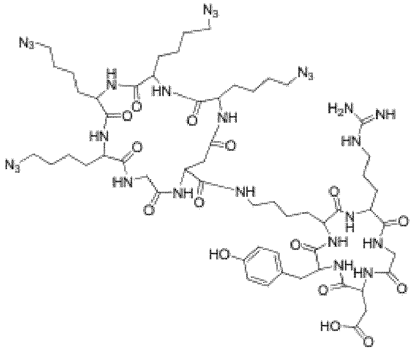

Targeting substance = cyclicRGDyK (9a), peptide A (9b), and binding group =

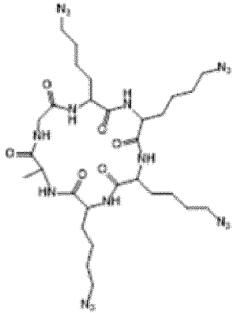

### Unit drug conjugate 10

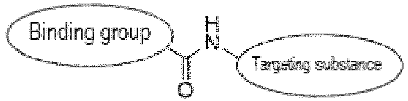

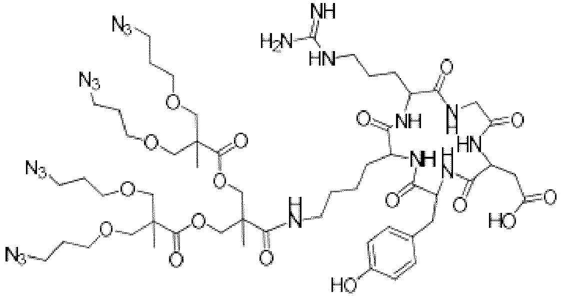

Targeting substance = cyclicRGDyK, and binding group =

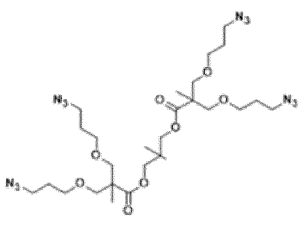

### Unit drug conjugate 11

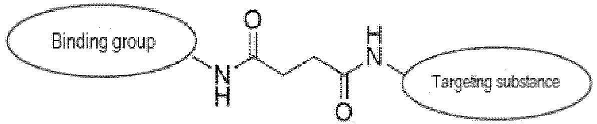

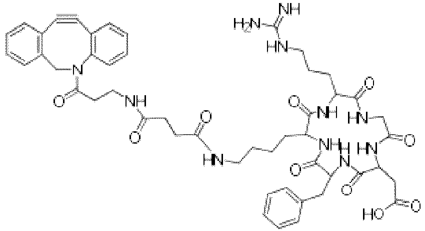

Targeting substance = cyclicRGDfK, and binding group = ADIBO

### Unit drug conjugate 12

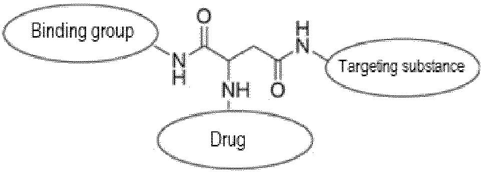

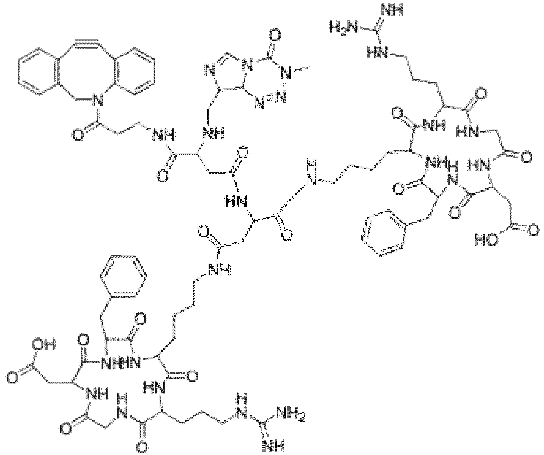

Targeting substance = peptide A, drug = temozolomide, and binding group = ADIBO

### Unit drug conjugate (12a-c)

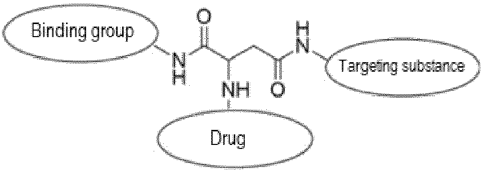

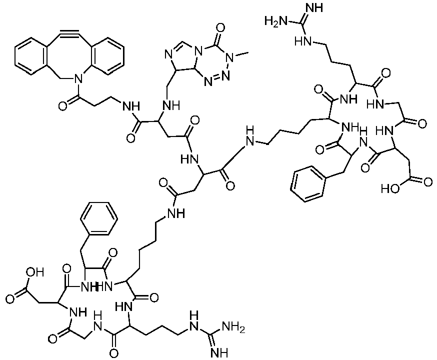

Targeting substance = peptide A, drug = temozolomide (12a), 5-fluorouracil (12b), sodium borocaptate (12c), and binding group = ADIBO

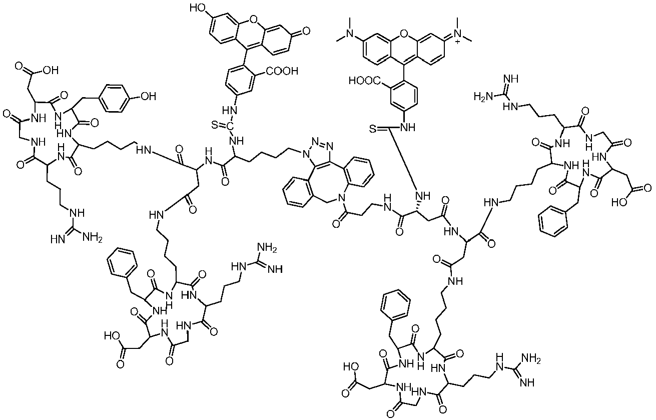

### Drug conjugate 1-2: a combination of unit drug conjugates 1 and 2

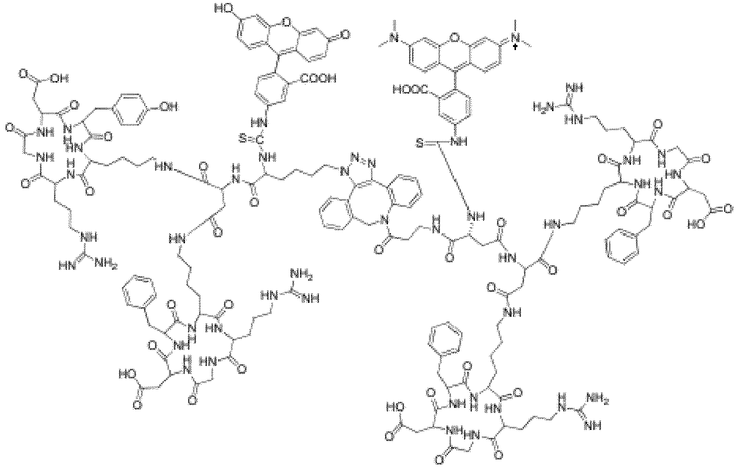

### Drug conjugate 3-4: a combination of unit drug conjugates 3 and 4

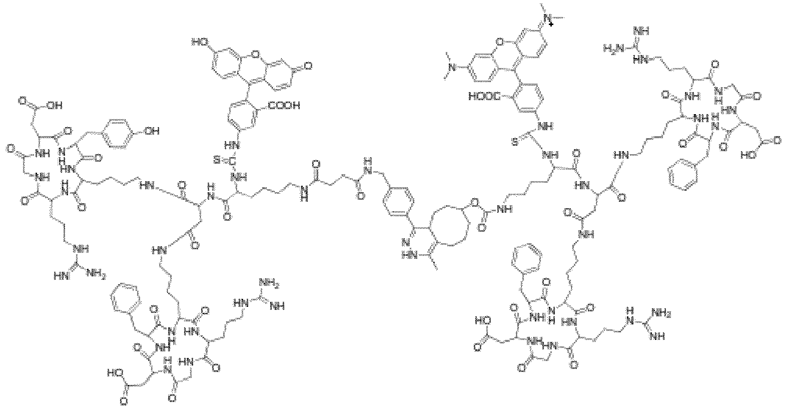

### Drug conjugate 5-6: a combination of unit drug conjugates 5 and 6

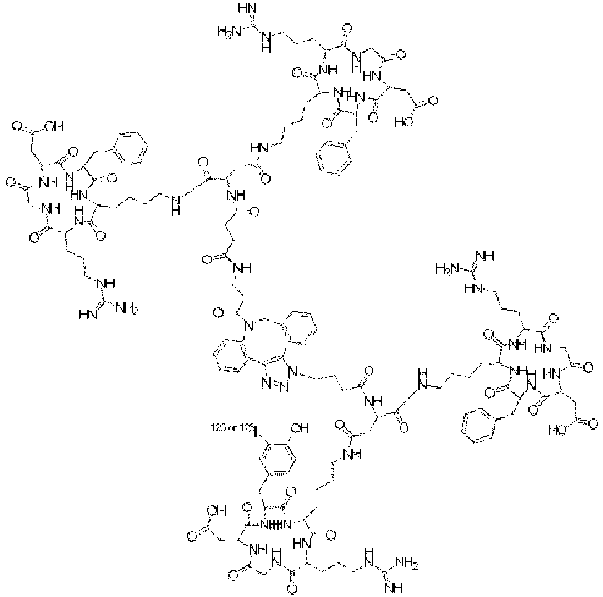

### Drug conjugate 6-7: a combination of unit drug conjugates 6 and 7

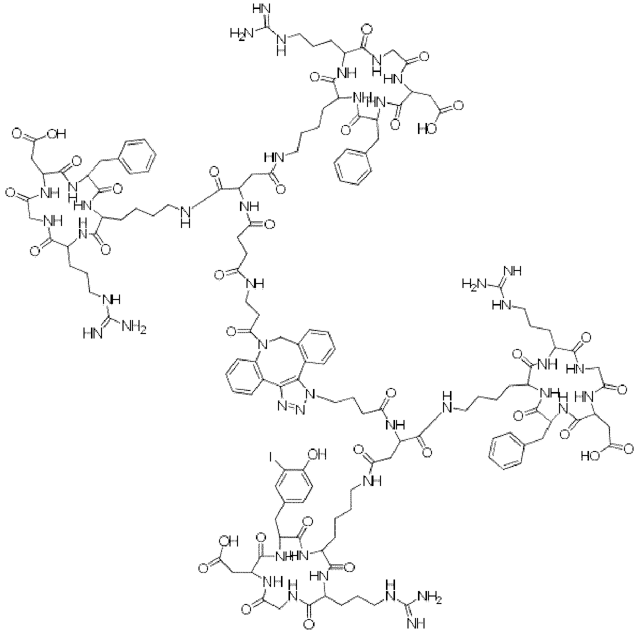

In another example, unit drug conjugates targeting PSMA were produced.

The selected targeting substance is a glutamate-urea-lysine (GLTL)-based motif that binds to PSMA, and the structure thereof is as follows. The targeting substance was synthesized based on Maresca K.P. et al., J Med Chem 2009; 52:347-357.

### Peptide C

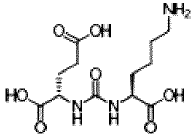

### Unit drug conjugate 13

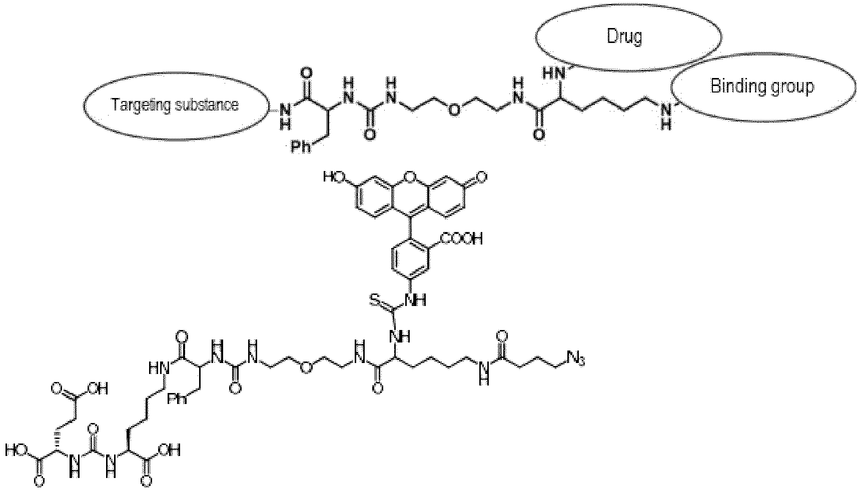

Targeting substance = peptide C, drug = FITC, and binding group = azide

### Unit drug conjugate 14

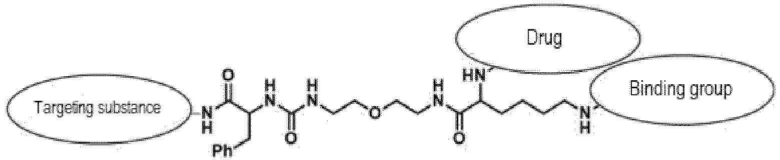

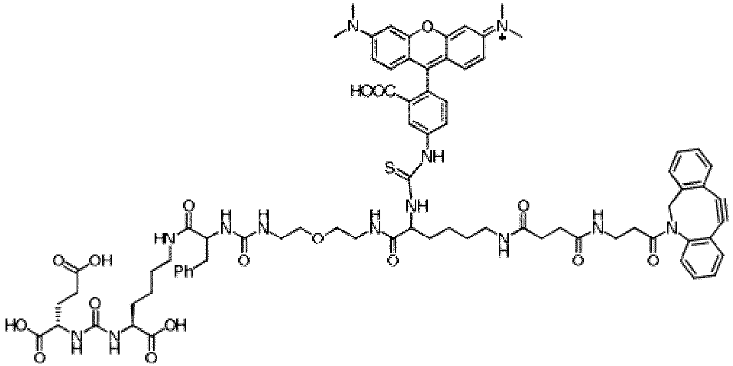

Targeting substance = peptide C, drug = TRITC, and binding group = ADIBO

### Unit drug conjugate 15

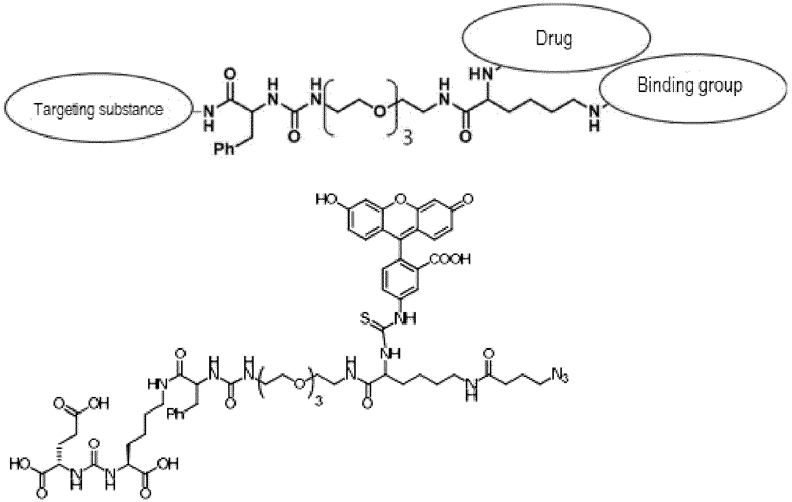

Targeting substance = peptide C, drug = FITC, and binding group = azide

### Unit drug conjugate 16

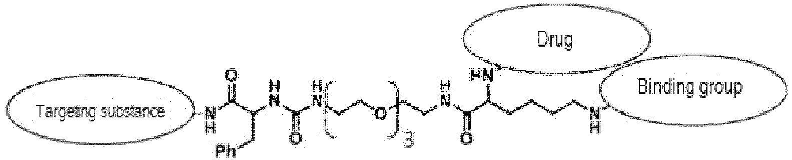

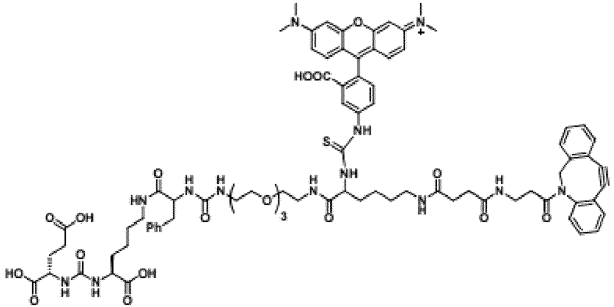

Targeting substance = peptide C, drug = TRITC, and binding group = ADIBO

### Unit drug conjugate 17

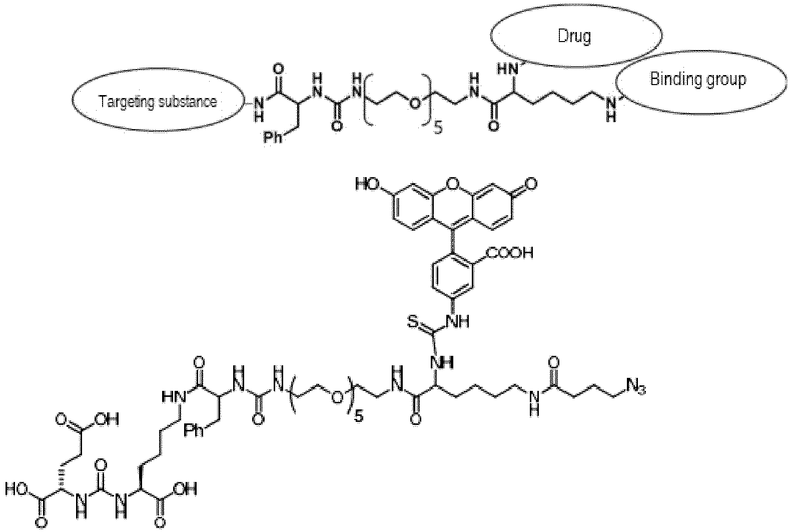

Targeting substance = peptide C, drug = FITC, and binding group = azide

### Unit drug conjugate 18

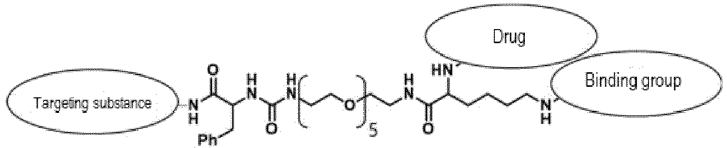

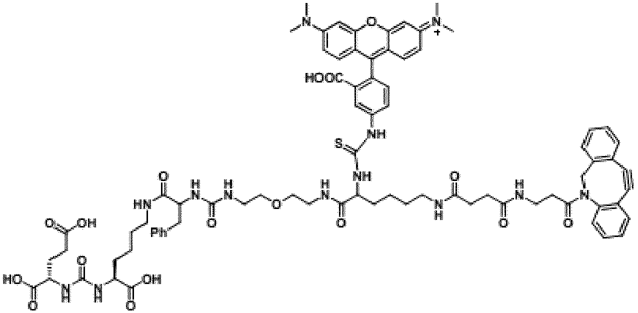

Targeting substance = peptide C, drug = TRITC, and binding group = ADIBO

### Example 2. Verification of crosslinking between unit drug conjugates

Unit drug conjugates 1 to 4 were used in the experiment in order to examine the time at which crosslinking and binding between the unit drug conjugates were completed under *in vitro* experimental conditions. Unit drug conjugate 1 was dissolved in a mixed solution of water and PBS buffer (v/v = 1:1, 0.5 mL), and then unit drug conjugate 2 dissolved in the same solution (0.5 mL) was added thereto and allowed to react at room temperature for 40 minutes. After starting the reaction, a small amount (0.1 mL) was collected from the reaction mixture every 10 minutes and analyzed using HPLC. The same experiment was also conducted on unit drug conjugate 3 and unit drug conjugate 4.

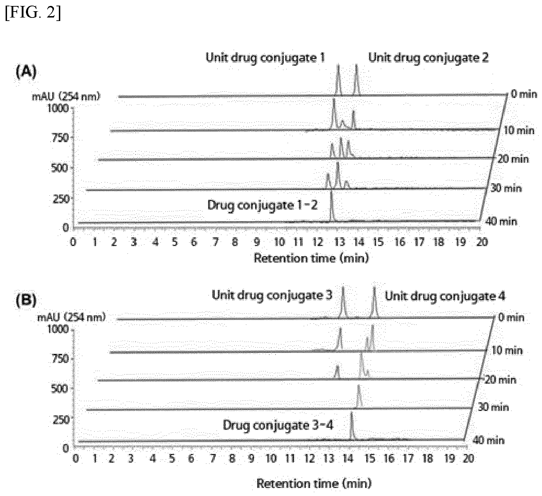

The analysis results are shown in FIG. 2, and it was confirmed that drug conjugate 1-2 by crosslinking between unit drug conjugates 1 and 2 was produced from 10 minutes after the start of the reaction, and the binding reaction was completed at 40 minutes, and that drug conjugate 3-4 by crosslinking between unit drug conjugates 3 and 4 was produced from 10 minutes after the start of the reaction, and the binding reaction was completed at 30 minutes. Thereby, it was experimentally demonstrated that, as originally designed, crosslinking between unit drug conjugates proceeds *in vitro* under conditions similar to *in vivo* conditions, indicating that there is no difficulty in proving the present invention in subsequent *in vitro* experiments.

### Example 3. Verification of in vivo binding affinity and cellular internalization of unit drug conjugates

The fluorescence resonance energy transfer (FRET) method is an analysis method that can examine the interaction between two compounds by measuring the fluorescence resonance energy between two fluorescent dyes occurring at 1 nm to 10 nm. The FRET method was used to measure the binding between the unit drug conjugates and the cellular internalization of the unit drug conjugates according to the present invention. The conditions of the FRET experiment for comparing and confirming cellular internalization were designed as shown in Table 1 below, and the unit drug conjugates used in this experiment were unit drug conjugates 1 and 2 and unit drug conjugates 3 and 4, respectively.

The post-conjugation targeting described in Table 1 below refers to a form in which unit drug conjugates are crosslinked before *in vitro* experiments. For example, it refers to drug conjugate 1-2 formed by binding between azide, which is the binding group of unit drug conjugate 1, and ADIBO, which is the binding group of unit drug conjugate 2, before targeting. The post-targeting conjugation refers to the technique according to the present invention, and in this technique, in the case of unit drug conjugates 1 and 2 as an example, unit drug conjugate 1 targets and binds to a target cell, and then unit drug conjugate 2 targets and binds to the same target cell. At this time, unit drug conjugate 1 and unit drug conjugate 2, which are a pair of unit drug conjugates, are crosslinked with each other by the binding groups thereof.

**[Table 1]**

| | Experimental Example 1 | Experimental Example 2 |
|---|---|---|
| | Treatment with unit drug conjugate 1 (50 | Treatment with unit drug conjugate 3 (50 µM) |
| 1 | µM) alone | alone |
| 2 | Treatment with unit drug conjugate 2 (50 µM) alone | Treatment with unit drug conjugate 4 (50 µM) alone |
| 3 | Treatment with unit drug conjugate 1 or 2 (50 µM) and target competitor (cyclic RGDyK, 300 µM) | Treatment with unit drug conjugate 3 or 4 (50 µM) and target competitor (cyclic RGDyK, 300 µM) |
| 4 | Treatment with drug conjugate 1-2 for post-conjugation targeting | Treatment with drug conjugate 3-4 for post-conjugation targeting |
| 5 | Post-targeting conjugation of unit drug conjugate 1 and unit drug conjugate 2 | Post-targeting conjugation of unit drug conjugate 3 and unit drug conjugate 4 |

1 × 10⁶ U-87MG cells (Korea Cell Line Bank) were seeded in a confocal dish (SPL Life science) and cultured at 37°C under 5% CO₂. The cells were treated with the unit drug conjugates alone or together with the target competitor according to the designed conditions 1 to 3 described in Table 1 above, and then binding to the cells was induced at 37°C for 2 hours. After the reaction, the cells were washed 3 times with 2 mL of PBS buffer and fixed in the culture dish using 4% paraformaldehyde for 1 hour at room temperature.

Fluorescence images were analyzed using a confocal laser scanning microscope (confocal microscopy, Nikon A1 Rsi), and Z-stack analysis was performed using NIS Elements Imaging software (version 5.01, NIKON).

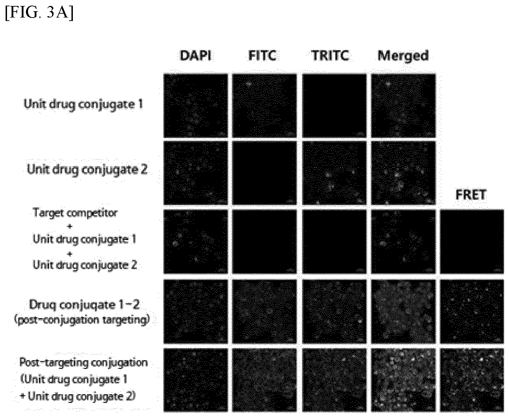

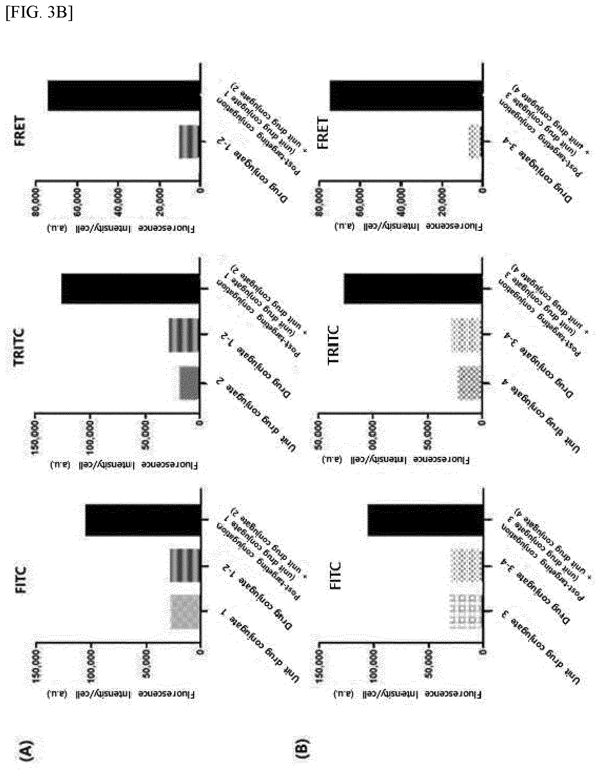

The fluorescence imaging results according to the experimental conditions described in Table 1 are shown in FIGS. 3A and 3B. FIG. 3A shows fluorescence images, and FIG. 3B depicts graphs showing the results of quantifying the fluorescence intensities of the fluorescence images. First, as a result of treating the cells with each of unit drug conjugates 1 or 2 alone, which can confirm the tumor cell uptake ability of each drug conjugate, the FITC channel and the TRITC channel, in which the wavelength of the fluorescent dye for each unit drug conjugate can be read, show that each unit drug conjugate is uptaken into the target cells. As a result of treating the cells with unit drug conjugate 1 together with the target competitor in order to confirm the selective binding affinity of the unit drug conjugate, no fluorescence signal could be observed in the FITC channel, indicating that unit drug conjugate 1 selectively bound to the target cells. Similarly, as a result of treating the cells with unit drug conjugate 2 together with the target competitor, no fluorescence signal was observed in the TRITC channel, indicating that unit drug conjugate 2 selectively bound to the target cells.

Meanwhile, in a control group of the present invention, before treatment of target cells, unit drug conjugates 1 and 2 were dissolved in ethanol and water (v/v = 1:1) and subjected to a binding reaction at room temperature for 40 minutes to synthesize drug conjugate 1-2 for post-conjugation targeting. Then, drug conjugate 1-2 was separated and cells were treated therewith. In an experimental group, target cells were treated sequentially with unit drug conjugates 1 and 2 for post-targeting conjugation. Thereafter, fluorescence wavelength analysis was performed in the FITC channel and TRITC channel, in which the wavelength of the fluorescent dye of each unit drug conjugate can be read, and the FRET channel in which the wavelength upon binding between the unit drug conjugates can be read.

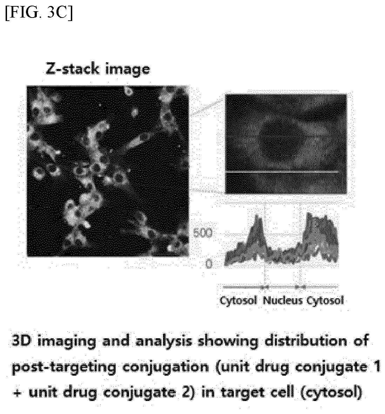

The analysis results are shown in FIGS. 3A and 3B. It was confirmed that, in the case of drug conjugate 1-2 for post-conjugation targeting, the intensity of fluorescence was measured to be quite low in the FRET channel, whereas when unit drug conjugate 1 was administered and then unit drug conjugate 2 was sequentially administered (i.e., post-targeting conjugation), the fluorescence intensity was 4 times stronger than when the cells were treated with drug conjugate 1-2 (i.e., post-conjugated target), indicating that endocytosis (cellular internalization) of the compound (unit drug conjugate) can be significantly improved when the unit drug conjugates capable of interacting with each other are sequentially administered. In addition, through Z-stack analysis that can analyze the fluorescence images three-dimensionally, it was confirmed that, when the cells were treated sequentially with the unit drug conjugates (post-targeting conjugation), the unit drug conjugates were distributed in the cytoplasm inside the cells (FIG. 3C).

The results of the experiment conducted according to Experimental Example 2 of Table 1 under the same conditions as the above-described experimental conditions are shown at the bottom of FIG. 3B, which shows results almost similar to those in Experimental Example 1. Thus, it could be confirmed once more that, when the unit drug conjugates are sequentially administered (i.e., post-targeting conjugation) according to the present invention, the effect of internalizing the compound into cells is significantly improved.

The principle of maximizing cellular internalization by sequentially introducing unit drug conjugates into cells according to the present invention is applicable to different types of drugs included in drug conjugates. In this case, the present invention is particularly advantageous for combination therapy in which various drugs must be administered together, and the present invention may be applied to multi-purpose therapy because it is possible to increase endocytosis of various drugs that are used for treatment of major diseases and complications.

### Example 4. Verification of target cell binding affinity, stability, and in vivo cellular internalization of unit drug conjugates

### 4-1. Stability test

The unit drug conjugates used in this experiment are unit drug conjugate 5 labeled with the radioisotope iodine, unit drug conjugate 6 not labeled with a radioisotope, and drug conjugate which is a combination of unit drug conjugates 5 and 6.

A stability test for unit drug conjugate 5 and drug conjugate 5-6 was performed using Radio-TLC. Serum obtained from human blood by centrifugation at 3,500 rpm for 5 minutes was used. 0.5 mL of the serum was treated with each of the compound unit drug conjugate 5 and drug conjugate 5-6 (3.7 MBq) labeled with a radioisotope. As a result of measuring stability using Radio-TLC (Bioscan) at five time points (10 minutes, 30 minutes, 60 minutes, 120 minutes, and 240 minutes) for 4 hours after treatment, it was shown that both drug conjugates showed stability of 90% or more, indicating that these drug conjugates in subsequent *in vivo* binding experiments (data not shown).

### 4-2. Binding affinity for target cells

The unit drug conjugates used in this experiment are unit drug conjugate 7 to which non-radioactive iodine has been introduced, and drug conjugate 6-7 which is a combination of unit drug conjugates 6 and 7.

The target cell binding affinities of unit drug conjugate 7 and drug complex 6-7 were measured using U-87MG cells (Korea Cell Line Bank), which are known to have high integrin αᵥβ₃ expression. The cells were treated with ¹²⁵I-c(RGDyV) (0.037 MBq) known as a competitive inhibitor, and were treated with different concentrations (0 to 5 nM) of each of unit drug conjugate 7 and drug conjugate 6-7 and cultured for 1 hour. Thereafter, the cells were precipitated by centrifugation and washed three times with PBS to remove unbound drug conjugate, and then the radiation dose was measured using a gamma counter.

The results of measuring the binding affinity (IC₅₀) of each drug conjugate are shown in Table 2 below.

**[Table 2]**

| No. | Drug conjugate | IC₅₀ (nM) |
|---|---|---|
| 1 | Peptide A | 343 |
| 2 | Peptide B | 337 |
| 3 | Unit drug conjugate 1 | 289 |
| 4 | Unit drug conjugate 2 | 208 |
| 5 | Unit drug conjugate 1-2 | 475 |
| 6 | Unit drug conjugate 7 | 1.08 ± 0.08 |
| 7 | Drug conjugate 6-7 | 0.52 ± 0.12 |
| 8 | Unit drug conjugate 12a | 580 |
| 9 | Unit drug conjugate 12b | 316 |
| 10 | Unit drug conjugate 12c | 310 |
| 11 | Unit drug conjugate 13 | 433.3 |
| 12 | Unit drug conjugate 14 | 186.6 |
| 13 | Drug conjugate 13-14 | 69.7 |

It was confirmed that the IC₅₀ of unit drug conjugate 7 was 1.08 ± 0.08 nM, and the IC₅₀ of drug conjugate 6-7 was 0.52 ± 0.12 nM. It was confirmed that both molecules showed a nanomolar (nM) level of binding affinity, indicating that unit drug conjugate 7 and drug conjugate 6-7 had excellent binding affinity to target cells.

### 4-3. Biodistribution Experiment in Tumor Model Mice

Unit drug conjugates used in this experiment are unit drug conjugate 5 labeled with the radioisotope iodine, unit drug conjugate 6 not labeled with a radioisotope, and drug conjugate 5-6 which is a combination of unit drug conjugate 5 and unit drug conjugate 6.

In this experiment, 1 × 10⁷ U-87MG cells were suspended in PBS and subcutaneously injected into mice (7 weeks old, BLAB/c nude mice, OrientBio, male) with congenital thymus defects, and then after a growth period of about 2 weeks, models with a tumor volume of 0.4 to 0.5 cc were selected and used.

Unit drug conjugate 5 (18.5 MBq) and drug conjugate 5-6 (18.5 MBq) were injected into the tail veins of the prepared nude mouse tumor models (n = 3, respectively), and 10 min, 30 min, 1 hour, and 2 hours, organs (blood, brain, heart, lung, liver, spleen, kidney, stomach, muscle, thigh, small intestine, large intestine, thyroid gland, tumor) were extracted and radiation dose was measured using a gamma counter (PerkinElmer, Wellesley, MA, USA).

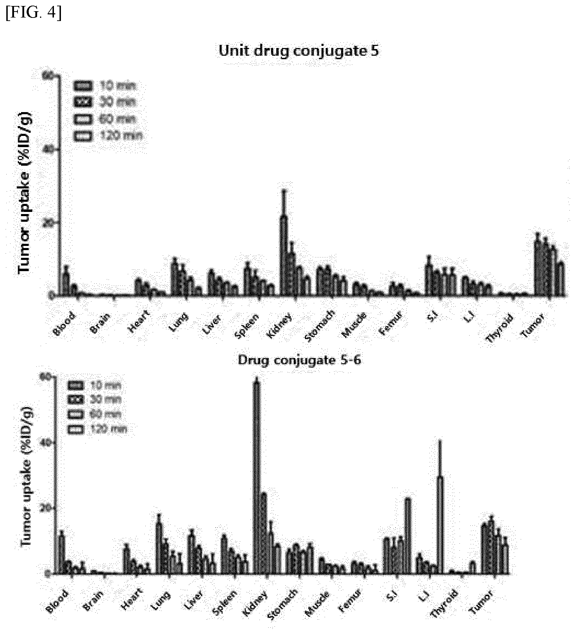

The results are shown in FIG. 4, and it was confirmed that both unit drug conjugate 5 and drug conjugate 5-6 show high uptake in the tumor at 15 minutes, but more than 50% thereof was discharged from the tumor within 2 hours and excreted mainly through the kidneys, and the uptake thereof in other organs was not high.

### 4-4. Comparison of enhancement of retention in tumor cells depending on dose of unit drug conjugate in tumor model mice

In the same manner as described in Example 4-3, 1 × 10⁷ U-87MG cells were suspended in PBS and subcutaneously injected into mice (7 weeks old, BLAB/c nude mice, OrientBio, male) with congenital thymus defects, and then after a growth period of about 2 weeks, models with a tumor volume of 0.4 to 0.5 cc were selected and used in the experiment. *In vivo* imaging was performed using SPECT/CT (NanoSPECT/CT, Bioscan Inc., Washington DC).

Unit drug conjugates 5 and 6 and drug conjugate 5-6 were used in the experiment. In a control, drug conjugate 5-6 (18.5 MBq) was injected (n = 3) or unit drug conjugate 5 (18.5 MBq) was injected (n = 3), and in an experimental group, unit drug conjugate 5 (18.5 MBq) was injected, and 15 minutes, and unit drug conjugate 6 was injected at doses of 18 mg/kg, 1.8 mg/kg, and 0.18 mg/kg (n = 3 for each dose). As described above, the experiment was conducted under a total of five conditions. 2 hours after administration of each unit drug conjugate (in the case of sequential administration, after the first administration), imaging was performed.

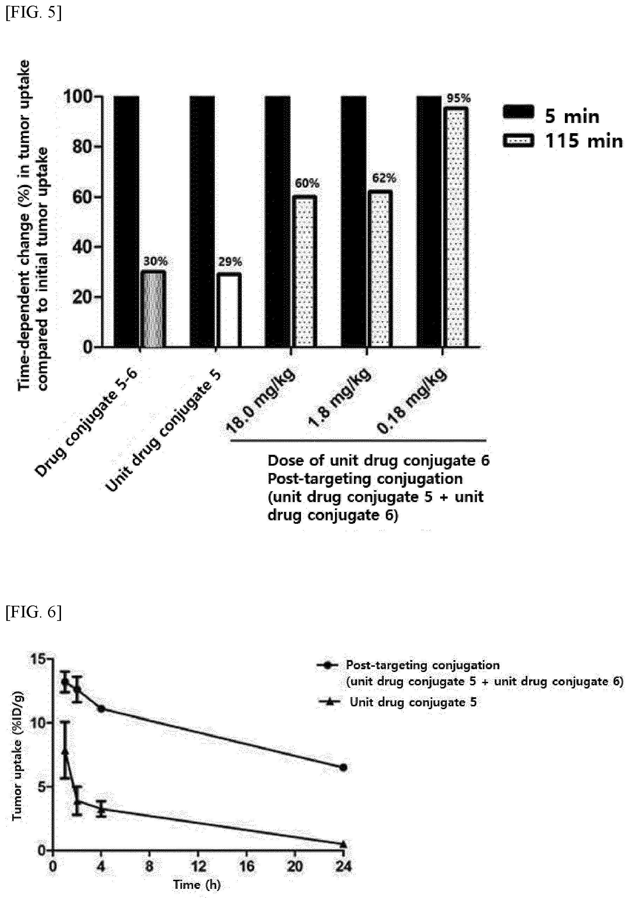

The results are in FIG. 5, and it was confirmed that, the control group, the tumor uptake of drug conjugate 5-6 and unit drug conjugate 5 decreased by less than half for 2 hours, whereas, in the experimental group treated sequentially with drug conjugates 5 and 6, more than 60% of tumor uptake remained for 2 hours regardless of the dose of unit drug conjugate 6. In particular, In the group sequentially treated with the smallest dose (0.18 mg/kg) of unit drug conjugate 6, more than 95% of tumor uptake was maintained up to 2 hours, and thus retention of the unit drug conjugate in the tumor was most prominent.

### 4-5. Comparison of retention enhancement effect in tumor cells for 24 hours in tumor model mice

In Example 4-4, the effect of enhancing retention in the tumor for a short time by sequential treatment with the unit drug conjugates was confirmed. Thus, an experiment was conducted to observe this effect for a longer period of time. To this end, the same tumor models as described in Example 4-3 were prepared, and on one hand, unit drug conjugate 5 (18.5 MBq) was administered to the tail vein of the nude mouse tumor models injected with U-87MG cells (n = 3), and on the other hand, unit drug conjugate 5 (18.5 MBq) and unit drug conjugate 6 (0.18 mg/kg) were sequentially administered at 15-minute intervals (n = 3). At 1 hour, 2 hours, 4 hours, and 24 hours after administration, the maintenance of uptake in the tumor and the half-life of each unit drug conjugate were compared by imaging. In addition, the tumor was excised at 10 minutes and 24 hours after administration of each unit drug conjugate, and the effect of enhancing retention in the tumor cells was evaluated *ex vivo.*

As a result, as shown in FIG. 6, it was confirmed that, when unit drug conjugate 5 was administered alone, the uptake thereof decreased to less than half of the initial intake within 4 hours, but when unit drug conjugates 5 and 6 were sequentially administered, the uptake thereof in the tumor was maintained at about 60% of the initial uptake up to 24 hours. It was confirmed that, when unit drug conjugate 5 was administered alone, the half-life thereof was 80.7 minutes, whereas when unit drug conjugates 5 and 6 were sequentially administered, the half-life thereof was 213.4 minutes, indicating that the half-life in the case of sequential administration was three times higher.

Meanwhile, as a result of evaluating the retention of the unit drug conjugate in tumor cells by extracting the tumor at 10 minutes and 24 hours after administration of the drug conjugate, it was confirmed that, when unit drug conjugate 5 (18.5 MBq, n = 3) was administered alone, the uptake thereof in the tumor was maintained at 10%, whereas when unit drug conjugate 5 (18.5 MBq, n = 3) and unit drug conjugate 6 (0.18 mg/kg) were sequentially administered, about 50% or more of the initial uptake thereof was maintained until 24 hours.

Taking the above results together, it can be seen that the drug conjugate according to the present invention can act on tumor cells for a long time after internalization into tumor cells.

### Industrial Applicability

The drug conjugates according to the present invention comprise a targeting substance that specifically binds to target cells, so that they may rapidly target the target cells. Together with this interaction between target cells and the targeting substance, binding groups allowing interaction between the drug conjugates are additionally introduced to the drug conjugates. Thus, when the drug conjugates are sequentially injected, the binding groups bind to each other *in vivo,* and *in vivo* crosslinking between complexes (different drug conjugates bound to the target cells) is induced, so that the drug conjugates form a kind of cluster. This clustering of the drug conjugates on the target cell surface artificially enhances cellular internalization caused by endocytosis, and thus the drug conjugates may exhibit maximized therapeutic and/or diagnostic effects even when administered at low doses and may prevent side effects caused by high-dose administration. Meanwhile, drugs introduced to the drug conjugates together with different binding groups may be designed in different ways, and thus co-administration of a diagnostic drug and a therapeutic drug or co-administration of a plurality of drugs exhibiting a synergistic effect is easy.

Although the present invention has been described in detail with reference to specific features, it will be apparent to those skilled in the art that this description is only of a preferred embodiment thereof, and does not limit the scope of the present invention. Thus, the substantial scope of the present invention will be defined by the appended claims and equivalents thereto.

## Claims

1. A unit drug conjugate wherein a binding group capable of binding to another unit drug conjugate is additionally linked to a drug conjugate in which a target substance, which binds specifically to target cells, and a drug, are linked together.

2. The unit drug conjugate according to claim 1, wherein the target substance and the drug are linked together by a linker.

3. The unit drug conjugate according to claim 2, wherein the binding group is additionally linked to the linker.

4. The unit drug conjugate according to claim 1, wherein the drug is a diagnostic drug or a therapeutic drug.

5. The unit drug conjugate according to claim 1, wherein the binding group is one or more.

6. The unit drug conjugate according to claim 1, wherein the drug is any one or more selected from the group consisting of maytansinoid, auristatin, aminopterin, actinomycin, bleomycin, talisomycin, camptothecin, N8-acetyl spermidine, 1-(2-chloroethyl)-1,2-dimethylsulfonyl hydrazide, esperamycin, etoposide, 6-mercaptopurine, dolastatin, tricotecene, calicheamycin, taxol, taxane, paclitaxel, docetaxel, methotrexate, vincristine, vinblastine, doxorubicin, melphalan, mitomycin A, mitomycin C, chlorambucil, duocarmycin, L-asparaginase, mercaptopurine, thioguanine, hydroxyurea, cytarabine, cyclophosphamide, ifosfamide, nitrosourea, cisplatin, carboplatin, mitomycin, dacarbazine, procarbazine, topotecan, nitrogen mustard, cytoxan, etoposide, 5-fluorouracil, bischloroethylnitrosourea (BCNU), irinotecan, camptothecin, bleomycin, idarubicin, daunorubicin, dactinomycin, plicamycin, mitoxantrone, asparaginase, vinorelbine, chlorambucil, melphalan, carmustine, lomustine, busulfan, treosulfan, decarbazine, etoposide, teniposide, topotecan, 9-aminocamptothecin, crisnatol, mitomycin C, trimetrexate, mycophenolic acid, tiazofurin, ribavirin, 5-ethynyl-1-beta-dribofuranosylimidazole-4-carboxamide (EICAR), hydroxyurea, deferoxamine, floxuridine, doxifluridine, raltitrexed, cytarabine (ara C), cytosine arabinoside, fludarabine, tamoxifen, raloxifene, megestrol, goserelin, leuprolide acetate, flutamide, bicalutamide, EB1089, CB1093, KH1060, verteporfin, phthalocyanine, photosensitizer Pe4, demethoxy-hypocrellin A, interferon-α, interferon-γ, tumor necrosis factor, gemcitabine, velcade, revamid, thalamid, lovastatin, 1-methyl-4-phenylpyridinium ion, staurosporine, actinomycin D, dactinomycin, bleomycin A2, bleomycinB2, peplomycin, epirubicin, pirarubicin, zorubicin, mitoxantrone, verapamil, thapsigargin, nuclease, toxins derived from bacteria or animals/plants, a radioisotope of ¹¹C, ¹⁸F, ⁹⁹mTc, ¹⁸⁸Re, ^{125/123/124/131}I, ⁸⁹Zr, ^{64/67}Cu, ⁶⁸Ga, ¹⁷⁷Lu, ⁹⁰Y, ²²⁵Ac or ²¹¹At, and fluorescein isothiocyanate (FITC), tetramethylrhodamine (TRITC), alexa fluor series or cyanine (Cy) series fluorescent dyes.

7. The unit drug conjugate according to claim 1, wherein the targeting substance binds to a target which is selected from the group consisting of integrin, prostate-specific membrane antigen (PSMA), CD3, CD4, CD6, CD11a, CD19, CD20, CD22, CD30, CD33, CD38, CD40, CD52, CD62, CD79b, CD80, CGRP, OX-40, CTLA4, 4-1BB, PD-1, EGF receptor, TNF receptor, Fc receptor, folate receptor, GD2, HER2, Her2/neu, HER3, HER4, VEGF receptor, interferon receptor, IgE receptor, IGF-1 receptor, interleukin 2 receptor, interleukin 5 receptor, interleukin 6 receptor, interleukin 17 receptor A, interleukin 31 receptor, interleukin 36 receptor, B7-H3, and CCR4, and which is expressed specifically on the target cells.

8. A drug conjugate comprising:
the unit drug conjugate (a first unit drug conjugate) of claim 1; and
another unit drug conjugate (a second unit drug conjugate) capable of binding to the unit drug conjugate of claim 1 by a binding group.

9. The drug conjugate according to claim 8, wherein the binding is binding by click reaction, binding by host-guest chemical interaction, or avidin-biotin binding.

10. The drug conjugate according to claim 9, wherein the binding by click reaction is azide-ADIBO binding, TCO-tetrazine binding, or alkyne-cyclopentadienone binding.

11. The drug conjugate according to claim 10, wherein the binding by host-guest chemical interaction is cucurbituril-adamantane binding or cyclodextrin-amino acid binding.

12. The drug conjugate according to claim 8, wherein the drug is a diagnostic drug or a therapeutic drug.

13. The drug conjugate according to claim 8, wherein the drug is one or more selected from the group consisting of maytansinoid, auristatin, aminopterin, actinomycin, bleomycin, talisomycin, camptothecin, N8-acetyl spermidine, 1-(2-chloroethyl)-1,2-dimethylsulfonyl hydrazide, esperamycin, etoposide, 6-mercaptopurine, dolastatin, tricotecene, calicheamycin, taxol, taxane, paclitaxel, docetaxel, methotrexate, vincristine, vinblastine, doxorubicin, melphalan, mitomycin A, mitomycin C, chlorambucil, duocarmycin, L-asparaginase, mercaptopurine, thioguanine, hydroxyurea, cytarabine, cyclophosphamide, ifosfamide, nitrosourea, cisplatin, carboplatin, mitomycin, dacarbazine, procarbazine, topotecan, nitrogen mustard, cytoxan, etoposide, 5-fluorouracil, bischloroethylnitrosourea (BCNU), irinotecan, camptothecin, bleomycin, idarubicin, daunorubicin, dactinomycin, plicamycin, mitoxantrone, asparaginase, vinorelbine, chlorambucil, melphalan, carmustine, lomustine, busulfan, treosulfan, decarbazine, etoposide, teniposide, topotecan, 9-aminocamptothecin, crisnatol, mitomycin C, trimetrexate, mycophenolic acid, tiazofurin, ribavirin, 5-ethynyl-1-beta-dribofuranosylimidazole-4-carboxamide (EICAR), hydroxyurea, deferoxamine, floxuridine, doxifluridine, raltitrexed, cytarabine (ara C), cytosine arabinoside, fludarabine, tamoxifen, raloxifene, megestrol, goserelin, leuprolide acetate, flutamide, bicalutamide, EB1089, CB1093, KH1060, verteporfin, phthalocyanine, photosensitizer Pe4, demethoxy-hypocrellin A, interferon-α, interferon-γ, tumor necrosis factor, gemcitabine, velcade, revamid, thalamid, lovastatin, 1-methyl-4-phenylpyridinium ion, staurosporine, actinomycin D, dactinomycin, bleomycin A2, bleomycinB2, peplomycin, epirubicin, pirarubicin, zorubicin, mitoxantrone, verapamil, thapsigargin, nuclease, toxins derived from bacteria or animals/plants, a radioisotope of ¹¹C, ¹⁸F, ⁹⁹mTc, ¹⁸⁸Re, ^{125/123/124/131}I, ⁸⁹Zr, ^{64/67}Cu, ⁶⁸Ga, ¹⁷⁷Lu, ⁹⁰Y, ²²⁵Ac or ²¹¹At, and fluorescein isothiocyanate (FITC), tetramethylrhodamine (TRITC), alexa fluor series or cyanine (Cy) series fluorescent dyes.

14. The drug conjugate according to claim 8, wherein the targeting substance binds to a target which is selected from the group consisting of integrin, prostate-specific membrane antigen (PSMA), CD3, CD4, CD6, CD11a, CD19, CD20, CD22, CD30, CD33, CD38, CD40, CD52, CD62, CD79b, CD80, CGRP, OX-40, CTLA4, 4-1BB, PD-1, EGF receptor, TNF receptor, Fc receptor, folate receptor, GD2, HER2, Her2/neu, HER3, HER4, VEGF receptor, interferon receptor, IgE receptor, IGF-1 receptor, interleukin 2 receptor, interleukin 5 receptor, interleukin 6 receptor, interleukin 17 receptor A, interleukin 31 receptor, interleukin 36 receptor, B7-H3, and CCR4, and which is expressed specifically on the target cells.

15. The drug conjugate according to claim 8, wherein a drug included in the first unit drug conjugate and a drug included in the second unit drug conjugate are different drugs.

16. A pharmaceutical composition for treating angiogenesis-related disease comprising the drug conjugate according to any one of claims 8 to 15.

17. The pharmaceutical composition according to claim 16, wherein the first unit drug conjugate and the second unit drug conjugate are sequentially administered.

18. The pharmaceutical composition according to claim 17, wherein the first unit drug conjugate is administered prior to the second unit drug conjugate, and the drug included in the unit drug conjugate is a radioisotope.

19. The pharmaceutical composition according to claim 16, wherein the angiogenesis-related disease is selected from the group consisting of benign tumor, malignant tumor (cancer), diabetic retinopathy, age-related macular degeneration, rheumatoid arthritis, endometriosis, psoriasis, chronic inflammation, coronary artery disease, atherosclerosis, stroke, ulcer, and myocardial infarction.

20. A composition for diagnosing angiogenesis-related diseases comprising the drug conjugate according to any one of claims 8 to 15.

21. The composition according to claim 20, wherein the first unit drug conjugate and the second unit drug conjugate are sequentially administered.

22. The composition according to claim 21, wherein the first unit drug conjugate is administered prior to the second unit drug conjugate, and the drug included in the first unit drug conjugate is a radioisotope.

23. The composition according to claim 20, wherein the angiogenesis-related disease is selected from the group consisting of benign tumor, malignant tumor (cancer), diabetic retinopathy, age-related macular degeneration, rheumatoid arthritis, endometriosis, psoriasis, chronic inflammation, coronary artery disease, atherosclerosis, stroke, ulcer, and myocardial infarction.

24. A pharmaceutical composition for diagnosing and treating angiogenesis-related disease comprising the drug conjugate according to any one of claims 8 to 15.

25. The pharmaceutical composition according to claim 24, wherein the first unit drug conjugate and the second unit drug conjugate are sequentially administered.

26. The pharmaceutical composition according to claim 24, wherein the angiogenesis-related disease is selected from the group consisting of benign tumor, malignant tumor (cancer), diabetic retinopathy, age-related macular degeneration, rheumatoid arthritis, endometriosis, psoriasis, chronic inflammation, coronary artery disease, atherosclerosis, stroke, ulcer, and myocardial infarction.
